# EUROPEAN PATENT SPECIFICATION

(11) **EP 1 150 703 B1**
(45) Date of publication and mention of the grant of the patent: **21.12.2005**
(21) Application number: 00903434.9
(22) Date of filing: 20.01.2000
(51) Int. Cl.: A61K 38/21, A61K 38/17, A61P 25/28

(54) **CHAPERONIN 10 AND BETA-INTERFERON THERAPY OF MULTIPLE SCLEROSIS**
CHAPERONIN 10 UND BETA-INTERFERON THERAPIE FÜR MULTIPLE SKLEROSE
TRAITEMENT DE LA SCLEROSE EN PLAQUES A LA CHAPERONINE 10 ET AU BETA-INTERFERON

(30) Priority: 20.01.1999 AU PP823999
(43) Date of publication of application: 07.11.2001
(73) Proprietor: THE UNIVERSITY OF QUEENSLAND, St Lucia, QLD 4072 (AU)
(72) Inventor: MORTON, Halle, Coorparoo Brisbane, QLD 4151 (AU); CAVANAGH, Alice, Mitchelton Brisbane, QLD 4053 (AU)
(74) Representative: Walton, Seán Malcolm
(86) International application number: PCT/AU2000/000032
(87) International publication number: WO 2000/043033

(56) References cited:
- AU-A- 1 103 495
- AU-A- 1 103 695

## Description

### FIELD OF THE INVENTION

THIS INVENTION relates to the treatment of Multiple Sclerosis using combined cpn10 and β-interferon therapy. This invention also relates to a pharmaceutical composition and kit comprising cpn10 and β-interferon

### BACKGROUND OF THE INVENTION

Multiple Sclerosis (MS) is the most common autoimmune disease involving the nervous system, affecting approximately 1 in 250,000 individuals in the United States. Clinically, MS is characterized by scattered central nervous system (CNS) demyelination leading to weakness, paresthesias, sensory loss and generally reduced control over neuromuscular function.

### Interferon-β (IFN-β).

IFN-β is an antiviral and antineoplastic cytokine produced by fibroblasts in response to viral stimulation. It acts as an immune system modulator and is the only treatment that has substantially altered the natural history of MS in a properly controlled clinical trial (The IFN-β Multiple Sclerosis Study Group, 1993, Neurology **43**:655-61). However, it was clear from this study that, while the high dose of IFN-β (8 million international units (MIU) injected every second day) was well tolerated and had a beneficial effect on the course of relapsing-remitting MS, it was only partially effective. These patients continued to have exacerbations, although at a reduced rate and of relatively mild clinical severity. However, this dose could not be increased, as an early pilot trial showed that increased doses (16 MIU), administered three times a week, produced unacceptable toxicity. Following administration of the two doses (1.6 and 8 MIU) used in the control clinical trial cited above, the incidence of notable side effects was low.

The most common laboratory abnormality associated with IFN-β treatment was lymphopenia unassociated with significant changes in the total white blood cell count. Other side effects noted were influenza-like symptoms and injection site reactions. *In vitro* studies have shown that IFN-β can effectively inhibit the proliferation of mitogen-stimulated peripheral blood mononuclear cells derived from both MS patients and healthy individuals. This anti-proliferative effect was associated with reduced IL-2R expression (Rudick *et al.,* 1993, Neurology, **43**:2080-7).

Extensive studies have been carried out in laboratory rats and mice to investigate the effect of IFN-β as a treatment of experimental autoimmune encephalomyelitis (EAE). EAE is the best available animal model of MS, and is a CD4⁺ T cell-mediated inflammatory demyelinating disease of the CNS in rodents (Pettinelli & McFarlin, 1981, J. Immunol. **127**:1420-3). EAE can be induced by inoculation of susceptible animals with CNS antigens (myelin basic protein, myelin proteolipid protein, myelin oligodendrocyte glycoprotein) and adjuvants. The development of signs of EAE is associated with the infiltration of the nervous system by T lymphocytes and macrophages (Raine, 1984, Lab. Invest. **50**:608-35). During spontaneous recovery from EAE there is a decline in the number of inflammatory cells in the nervous system (McCombe *et al.,* 1992, J. Neurol. Sci. **113**:177-86), due in part to apoptosis of T lymphocytes and macrophages in the central nervous system (Tabi *et al.,* 1994, Eur. J. Immunol. **24**:2609-17; McCombe *et al.,* 1996, J. Neurol. Sci., **139**:1-6). Recovery from EAE is also associated with production of downregulatory cytokines such as IL-10 and TGF-β (Kennedy *et al.,* 1992, J. Immunol. **149**:2496-2505).

In studies by Ruuls *et al.*, 1996, J. Immunol. **157**:5721-31), EAE was induced in Lewis rats by inoculation with a synthetic MBP peptide, residues 63-88. Treatment of these rats with recombinant rat IFN-β (3 x 10⁵ U/day s.c.) from day 8 to day 17 after inoculation fully protected the animals from disease during the treatment period. However, with cessation of treatment, most animals developed a protracted and remitting disease course with strongly enhanced severity. If treatment was continued to day 26, the animals did not relapse after treatment was discontinued. From these results, it would appear that discontinuation of treatment during the recovery phase of EAE is associated with rapid onset of severe paralytic disease.

One of the characteristic features of EAE is progressive weight loss during the clinical phase of the disease, which is rapidly reversed when the animals recover (Ruuls *et al*., 1996, *supra*). Treatment with IFN-β from day 8 to day 26 inhibited weight reduction, when compared with the control group. However, subsequent to treatment, all animals showed retardation of growth, which became evident approximately 25 days after inoculation. Failure to gain weight has also been observed as one of the most common and dose-limiting side effects of patients during IFN-β treatment for various diseases.

Using a mouse model of EAE, Yu *et al*., 1996, J. Neuroimmunol. **64**:91-100 determined the effect of treatment with mouse IFN-β on the course of EAE in (SWR x SJL)F₁ mice, following immunization with the myelin proteolipid protein (PLP) immunodominant peptide p139-151. They showed a marked decrease in mean neurological deficit, a significant delay in exacerbation onset and exacerbation rate and a decrease in DTH. IFN-β treatment produced a long-term improvement in mean clinical score, a clear histopathological improvement and a delay in progression to disability. *In vitro,* IFN-β inhibited the proliferation of determinant-primed lymph node cells in a dose dependent manner. In conclusion, these authors noted that there was a clear histopathological improvement in the IFN-β-treated mice but that the improvement was far from a cure. A similar significant but modest long-term therapeutic effect on severity and incidence of CNS inflammation has been described in MS patients (IFN-β Multiple Sclerosis Study Group, 1993).

### Early pregnancy factor (EPF).

EPF was first described in Morton *et al.,* 1974, Nature, **249**:459-60 and Morton *et al.,* 1976, Proc. R. Soc. Lond., **193**:413-9.

EPF appears in maternal serum within 6-24 hr of fertilization, is present for at least the first half of pregnancy in all species studied and is essential for continued embryonic growth and survival (Morton *et al.,* 1987, Current Topics in Developmental Biology **23**:73-92; Athanasas-Platsis *et al.,* 1989, J. Reprod. Fert. **87**:495-502; Athanasas-Platsis *et al.,* 1991, J. Reprod. Fert. **92**:443-51). EPF is also an autocrine survival factor for tumour cells (Quinn *et al.,* 1990, Clin. Exp. Immunol. **80**:100-8; Quinn & Morton, 1992, Cancer Immunol. Immunother. **34**:265-71) and for regenerating liver cells following partial hepatectomy (Quinn *et al.,* 1994, Hepatology **20**:1294-302). Like many other growth factors, EPF is present in platelets suggesting that it may have a physiological role in wound healing (Cavanagh & Morton, 1994, Eur. J. Biochem. **222**:551-60).

EPF also has immunomodulatory properties. This was first proposed when it was shown that EPF is able to augment the rosette-inhibiting properties of an immunosuppressive anti-lymphocyte serum (Morton *et al.,* 1974, *supra;* Morton *et al.,* 1976, *supra*), and of anti-CD4 but not anti-CD8 antibodies (Morton *et al.,* 1982, Pregnancy Proteins, Eds. B. Grudzinskas, B. Teisner, M. Seppala, Academic Press, Sydney, 391-405). Further studies showed that EPF can suppress the delayed-type hypersensitivity (DTH) reaction to trinitrochlorobenzene (TNCB) in mice (Noonan *et al.,* 1979, Nature **278**:649-51), suppress mitogen-induced lymphocyte proliferation (Athanasas-Platsis, 1993, PhD Thesis, The University of Queensland) and suppress IFN-γ production by EPF-binding T cells. The immunosuppressive action of EPF is mediated through the sequential induction of suppressor factors and/or lymphokines. EPF binds to CD4+ T cells, sequentially releasing two genetically-restricted suppressor factors EPF-S₁ and EPF-S₂ (Rolfe *et al,* 1988, Clin. Exp. Immunol. **73**:219-25; Rolfe *et al*, 1989, Immunol. Cell Biol. **67**:205-8). While EPF is neither species- nor strain-restricted in its action, EPF-S₁ activity is restricted to the I region of the mouse MHC and HLA-DR in human while EPF-S₂ action is localized to the lgh region.

### Chaperonin 10

Amino acid sequencing of EPF purified from human platelets (Cavanagh & Morton, 1994, *supra*) has shown it to share amino acid sequence with chaperonin 10 (cpn10), a member of the heat shock family of proteins (Hartman *et al*., 1992, Proc. Natl. Acad. Sci. U.S.A. **89**:3394-8). Thus EPF and cpn10 appear to be homologs. Cpn10 is present in a variety of organisms from bacteria to humans. The structure of cpn10 is highly conserved across mammalian species, although less so between bacteria and mammals. Cpn10 is present in mitochondria where it has a role in protein folding (Ellis & van der Vies, 1991, Annu. Rev. Biochem. **60**:321-47), and like other heat shock proteins, cellular cpn10 is upregulated during cellular stress.

Like EPF, cpn10 is active in rosette-inhibition assays and displays immunosuppressive activity such as evidenced by prolonging the viability of allogeneic skin grafts in rats. With regard to EAE, reference is made to International Publication No. WO95/15338, wherein cpn10 was shown to delay the onset of EAE and modify the clinical features of the disease in a rat model.

Thus, it is apparent from the literature that both cpn10 and IFN-β are immunosuppressive.

### OBJECT OF THE INVENTION

In subsequent experiments, the present inventors have compared cpn10 and INF-β with respect to treating EAE in mice, and have surprisingly discovered that cpn10 and INF-β act via different mechanisms. As a result of this discovery, it has now been realized that cpn10 and INF-β can co-operate with each other to provide an improved treatment of EAE. The implication is therefore that cpn10 and INF-β may act co-operatively to provide an improved treatment of MS in humans.

It is therefore an object of the invention to provide pharmaceutical composition for treating Multiple Sclerosis (MS) and its use.

### SUMMARY OF THE INVENTION

In a first aspect, the present invention resides in the claim 1.

In a preferred embodiment, the invention is used to prevent MS relapse.

In a second aspect, there is provided a pharmaceutical composition for the treatment of MS comprising a pharmaceutically-effective amount of cpn10 and INF-β and a pharmaceutically-acceptable carrier or diluent.

In a third aspect, there is provided a kit comprising a pharmaceutical-effective amount of cpn10 and IFN-β together with a pharmaceutically-acceptable carrier or diluent.

Preferably, said IFN-β is in dehydrated form which in use, is rehydrated by said pharmaceutically-acceptable carrier or diluent.

In one embodiment, said cpn10 is in dehydrated form and in use, is rehydrated by said pharmaceutically-acceptable carrier or diluent.

In another embodiment, said cpn10 is in tablet or capsule form.

According to the aforementioned aspects, preferably the pharmaceutically-effective amount of cpn10 and IFN-β comprises 5-60 mg of cpn10.

More preferably, the pharmaceutically-effective amount of cpn10 and IFN-β comprises 10-30 mg of cpn10.

Preferably, the pharmaceutically-effective amount of cpn10 and IFN-β comprises 1-10 MIU of IFN-β.

More preferably, the pharmaceutically-effective amount of cpn10 and IFN-β comprises 4-6 MIU of IFN-β.

Throughout this specification and claims which follow, "*comprise*"*,* "*comprises*" and "*comprising*" are used inclusively rather than exclusively, whereby a stated integer or group or integers may include one or more non-stated integers or groups of integers.

### BRIEF DESCRIPTION OF THE FIGURES AND TABLES

**FIG. 1**
   Recombinant cpn10 administered intraperitoneally to EAE rats commencing day of innoculation with MBP. Values represent mean disability ± standard error of the mean (SEM). Cpn10 treatment significantly decreased the total disability score (p = 0.001; Mann-Whitney Rank Sum Test, Sigma Stat 2.0, Jandel Scientific).
**FIG. 2**
   Recombinant cpn10 administered orally to EAE rats commencing day of innoculation with MBP. Values represent mean disability ± standard error of the mean (SEM). Cpn10 treatment significantly decreased the total disability score (p = 0.005; Mann-Whitney Rank Sum Test, Sigma Stat 2.0, Jandel Scientific).
**FIG. 3**
   Recombinant cpn10 pGEX administered intraperitoneally (i/p) or orally (O/F) to EAE rats commencing day of inoculation with MBP. Values represent % weight loss per group (compared to weight at day 10).
**FIG. 4**
   Delayed type hypersensitivity (DTH) reaction to MBP in rats, administered with recombinant cpn10 intraperitoneally (ip) or orally, or treated with control vehicle, commencing day of innoculation with MBP. Four groups of rats (n=4 per group) were inoculated with MBP on day 0 and received cpn10 (50 µg/rat) by oral feeding (O/F; group 2) or ip (group 3) or vehicle alone ip (groups 1 and 4) daily from day 0 to 16. On day 15, ear thickness of both ears of rats was measured, then 20 µg MBP in 10 µl saline was injected into the base of both ears in groups 2-4. Group 1 received injections of saline. After 24 hr, both ears were again measured and ear swelling determined by subtraction. *** p<0.001; **p=0.003 compared to group 4 (Student's *t* test).
**FIG. 5**
   Effect of recombinant cpn10 administered i.p daily or orally to rats (n=3), commencing day of innoculation with MBP (day 0), on total and differential leukocyte counts. Counts (shown with standard deviations) were obtained on day 16.
**FIG. 6**
   Suppression of MBP-stimulated lymphocyte proliferation *in vitro* by recombinant cpn10. Lymphocytes were recovered from draining lymph nodes of rats 10 days after innoculation with MBP. Dilutions of cpn10 were prepared to give a final concentration on the plate and 100 µl dispensed in triplicate. To each well was added 50 µl washed lymphocytes (4 x 10⁶/ml) and 50 µl MBP (80 µg/ml). Control wells contained either: a) cells with MBP, no cpn10, or b) cells with no MBP, no cpn10. Plates were incubated for 72 hr and pulsed during the last 18 hr with 0.5 µCi [*methyl*-³H] thymidine. Proliferation was assessed by measuring incorporated radioactivity (cpm + standard deviations) in cells incubated with or without cpn10. ***p<0.001; **p=0.005 (Student's test).
**FIG. 7**
   Decrease in mean disability score of EAE in SJL mice treated with recombinant cpn10 compared with mice treated with vehicle alone. EAE was induced in mice (n=10 per group) with PLP peptide p139-151 and cpn10 (10 µg/mouse/48 hr or 2.5 µg/mouse/48 hr) or vehicle alone administered ip from day 0 to day 20. From day 8, clinical signs were monitored daily and total disability of each mouse recorded. The results are expressed as mean disability score/group.
**FIG. 8**
   Mean disability score of PLP-EAE in SJUJ mice (± sem) treated with **a.** recombinant cpn10 (rcpn10) or rcpn10+IFN-β, **b.** IFN-β or rcpn10+IFN-β, compared with mice treated with vehicle alone. EAE was induced by inoculation with PLP peptide on d 0 and mice (n = 10/group) received a. rcpn10 (2.5 µg/mouse) ip every other day from d 0 to d 20, **b.** IFN-β (5 x 10³ units/mouse) sc every other day from d 10 to d 20 or a combination of both. Control mice received vehicle alone. From d 8 to d 60, clinical signs were monitored daily and mean disability score of each group recorded. The results are expressed as mean disability score per group.

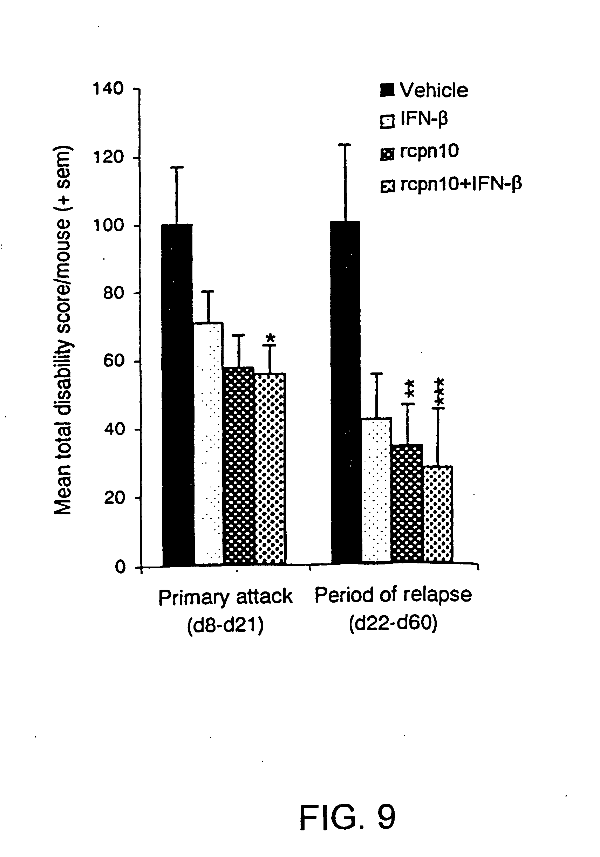

**FIG. 9** Total mean disability score (± sem) of PLP-EAE in SJUJ mice, treated with recombinant cpn10, IFN-β or a combination of both, during the primary attack and period of relapse. EAE was induced by inoculation with PLP peptide on d 0 and mice were treated with rcpn10 ip (2.5 µg/mouse every other day from d 0 to d 20), IFN-β *sc* (5,000 units/mouse every other day from d 10 to d 20) or a combination of both. Control mice received vehicle alone. Clinical signs were monitored from d 8 to d 21 and from d 22 to d 60. Combined treatment with rcpn10 and IFN-β significantly suppressed disability during the primary attack compared with the control group (*p = 0.050) as did rcpn10 or rcpn10+IFN-β during the period of relapse (**p=0.030, ***p=0.014).

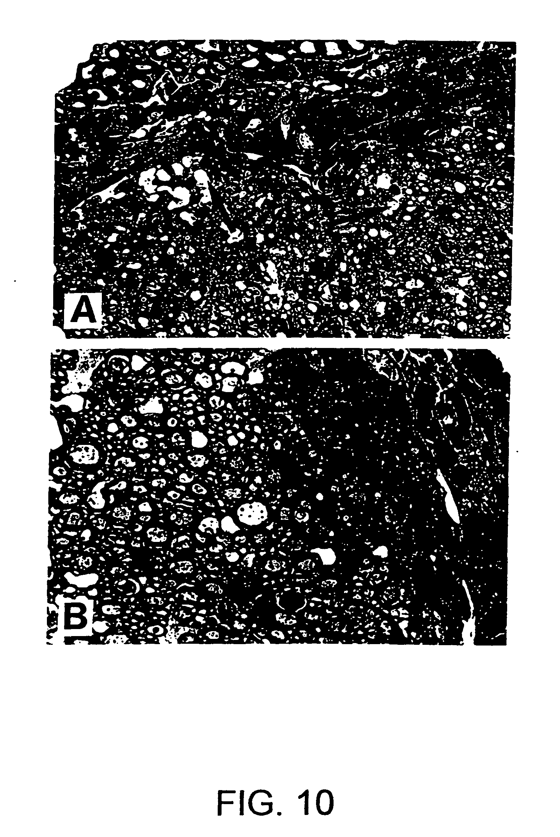

**FIG. 10** Sections from the sacral spinal cord of (A) control mouse and (B) mouse treated with recombinant cpn10 (2.5 µg/mouse, *ip* every other day from d 0 to d 20), sampled on d 14. In the control mouse (A) there was inflammation (large arrow) and prominent areas of demyelination (small arrows) of many fibres. In the cpn10-treated mouse (B), inflammation (large arrow) in the subpial regions was observed with little demyelination evident.

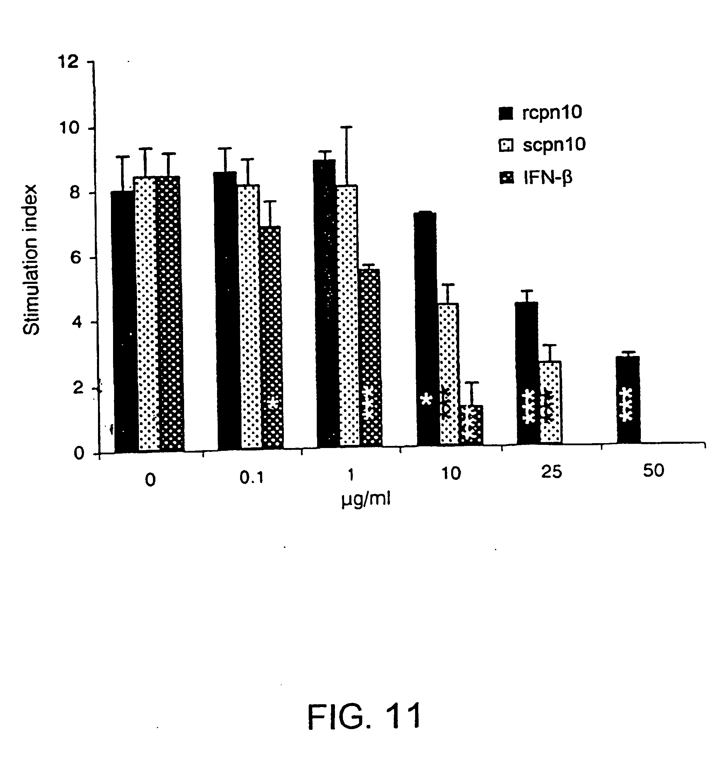

**FIG. 11** Suppression of MBP-stimulated lymphocyte proliferation *in vitro* by rcpn10, synthetic cpn10 (scpn10) and IFN-β. Lymphocytes were recovered from draining lymph nodes of rats 10 days after inoculation with MBP and incubated (2 x 10⁵ cells) with MBP (20 µg/ml) and rcpn10, scpn10 and IFN-β at the concentrations shown. Control wells contained either: a) cells with MBP, no cpn10 or b) cells with no MBP, no cpn10. Plates were incubated for 3 days and pulsed during the last 18 h with 0.5 µCi [*methyl*-³H]thymidine. Proliferation was assessed by radioactivity incorporated into the cells and was expressed as stimulation index (mean of wells with MBP/mean of wells without MBP; standard deviations are indicated). Results in wells with cpn10 or IFN-β were compared with those in wells without cpn10 or IFN-β. ***p <0.001, *p = 0.05 (Student's *t* test).

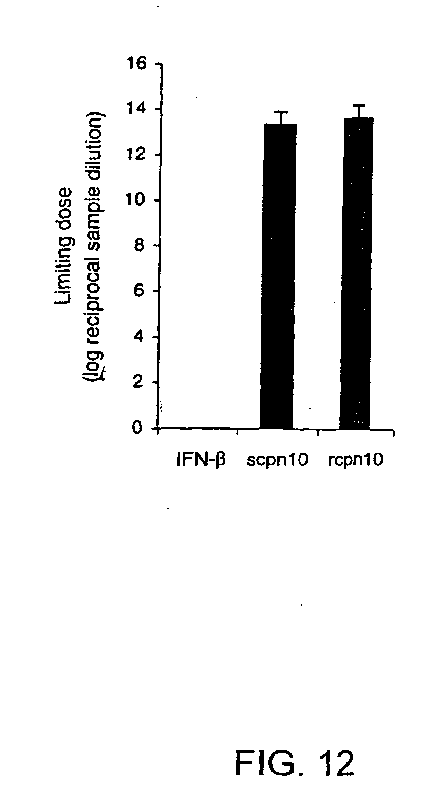

**FIG. 12** Activity of IFN-β, synthetic cpn10 (scpn10) and recombinant cpn10 (rcpn10) in the rosette inhibition test. Cpn10 preparations (50 µg/ml) and IFN-β (0.5 µg/ml) were diluted 10-fold and the rosette inhibition titre of each dilution was determined. The limiting dose (log reciprocal sample dilution, with standard deviation indicated) was recorded as the highest dilution of a sample to give a positive result in the assay.
**TABLE 1**
   List of IFN-β preparations currently used in the treatment of MS. *sc* = subcutaneous injection; *im* = intramuscular injection.
**TABLE 2**
   Treatment with recombinant cpn10 decreases mean total clinical score of MBP-EAE in Lewis rats. Rats were inoculated with MBP in one rear footpad on day 0. cpn10 or vehicle alone were administered intraperitoneally (ip) or orally (7 rats per group) at the time intervals indicated commencing day 0 until day 17. Clinical signs were recorded daily (see methods) from day 10 to day 20, and examination of these groups was continued to day 35 (indicated thus *) and the mean clinical score per rat determined for each group. Mean total clinical score per rat in the test group was compared with the mean total clinical score per rat in the group receiving vehicle alone, tested concurrently (Mann-Whitney Rank Sum Test).
**TABLE 3**
   Treatment with rcpn10 decreases the total disability score of PLP-EAE in SJL/J mice. EAE was induced in SJL/J mice with PLP peptide 139-151 (Greer *et al.,* 1996, J. Immunol. **156**: 171-179). Mice (10 per group) were treated with rcpn10 or vehicle and were clinically assessed from day 8 to day 42 or day 60. Results were recorded as total mean disability score/mouse over the examination period in the rcpn10 treatment group compared with that in the group receiving vehicle alone tested concurrently (Mann-Whitney Rank Sum test).
**TABLE 4**
   Combination treatment with recombinant cpn10 (rcpn10) and IFN-β decreased the total clinical score of PLP-EAE in SJUJ mice. EAE was induced in SJUJ mice with PLP peptide 139-151. Mice (n=10 per group) were treated with rcpn10, IFN-β or both in combination, or with vehicle alone as described previously. Mice were clinically assessed from day 8 to day 60, from day 8 to day 21 and from day 22 to day 60. Results were recorded as total mean clinical score per mouse in each group over the periods of examination. Results in the rcpn10, IFN-β and rcpn10 + IFN-β treatment groups were compared with those in the group receiving vehicle alone tested concurrently (Mann-Whitney Rank Sum Test).
**TABLE 5**
   Prevention of antigen-induced anaphylactic death in SJL/J mice. EAE was induced in 4 groups of SJUJ mice with PLP peptide 139-151 on day 0. On day 0, Groups 1 and 2 received 2.5 µg cpn10 per mouse/48 hr ip, while Groups 3 and 4 received vehicle alone. On day 10, Group 1 and Group 3 received 0.5 x 10⁴ units mouse IFN-β in PBS/0.1% BSA and Groups 2 and 4 received PBS/BSA. From day 8, mice were weighed and examined daily for clinical signs of EAE. The data show the number of mice in each group that died 8 to 12 days after commencement of treatment with IFN-β in PBS/0.1 % BSA or PBS/BSA alone, as a fraction of the total mice in the respective groups. A statistical comparison of death rates between Groups 1 and 2 v Groups 3 and 4 revealed a p value <0.02 using Chi-square analysis.

### DETAILED DESCRIPTION OF THE INVENTION

It will be appreciated that the present invention has arisen, at least in part, from the discovery by the present inventors that cpn10 and IFN-β act via distinct mechanisms to co-operatively relieve mice and rats of EAE symptoms. In particular, combined IFN-β and cpn10 treatment displayed a markedly improved ability to prevent EAE relapse following cessation of treatment. Accordingly, this discovery has implications for the treatment of MS in humans, EAE being the best experimental animal model of human MS. In this regard, administration of IFN-β is a well-known treatment of MS, but produces side effects, particularly at higher doses of IFN-β. Thus, the present invention provides a combination therapy wherein cpn10 and IFN-β provide greater relief from disease symptoms than does IFN-β alone, and thereby reduces the need for IFN-β to be administered at doses which produce side-effects. It should also be appreciated that IFN-β has become such an indispensable treatment for MS that combined therapy using cpn10 and IFN-β, will be extremely attractive in that patients need not be removed from IFN-β treatment, or can be removed with reduced likelihood of MS relapse.

In a first aspect, the present invention therefore provides the claim 1.

In a preferred embodiment, the invention is used to prevent MS relapse.

As used herein "*MS relapse*" is the recurrence of MS symptoms after recovery from primary attack. MS relapse can occur, for example, if an individual is taken off IFN-β therapy or the amount of IFN-β is reduced due to the risk or appearance of side-effects. The method of the invention may prevent MS relapse, in that re-appearance of MS symptoms may be delayed, or in that the symptoms are reduced in severity.

By "*pharmaceutically-effective amount*" is meant an amount of medicament or drug, for example cpn10 and IFN-β, which when administered to an individual, prevents, alleviates, reduces or eliminates disease symptoms in said individual, and/or reduces the severity or duration of said disease symptoms. It will also be understood that the present invention contemplates administration of cpn10 and IFN-β in amounts where alone, cpn10 or IFN-β might be ineffective or sub-optimal, but in combination, are pharmaceutically-effective.

Preferably, the pharmaceutically-effective amount of cpn10 and IFN-β comprises 5-60 mg of cpn10.

More preferably, the pharmaceutically-effective amount of cpn10 and IFN-β comprises 10-30 mg of cpn10.

Preferably, the pharmaceutically-effective amount of cpn10 and IFN-β comprises 1-10 million International Units (MIU) of IFN-β.

More preferably, the pharmaceutically-effective amount of cpn10 and IFN-β comprises 4-6 MIU of IFN-β.

It will be appreciated by the skilled person that the aforementioned pharmaceutically-effective amounts are calculated in terms of a typical 70 kg individual. Accordingly, doses may vary depending on the weight, age, sex, general health and fitness of the individual and any other treatments to which the individual is being subjected. Furthermore, the amount of cpn10 and IFN-β administered will be interdependent with the frequency and timing of administration.

In this regard, it is contemplated that the frequency and timing of administration of cpn10 and IFN-β may vary. Accordingly, it will be apparent that there are two modes of treatment contemplated within the scope of the invention:-
(i) combined IFN-β and cpn10 treatment wherein each is administered at different times and/or with different frequencies; and
(ii) treatments where IFN-β and cpn10 are administered together.

Preferably, cpn10 is to be administered daily, although administration on a less frequent basis (e.g. twice or thrice weekly) is also possible.

As will be discussed hereinafter, IFN-β is usually administered once weekly or thrice weekly, depending on the source of IFN-β. Such administration frequencies could be maintained irrespective of how frequently cpn10 is administered. However, it is preferable that IFN-β and cpn10 are administered together daily. This latter mode of administration would be particularly suited where cpn10 and IFN-β are combined in said pharmaceutical composition for subcutaneous or intramuscular injection.

In an embodiment, IFN-β is to be administered by subcutaneous or intramuscular injection and cpn10 is administered by injection or orally.

Preferably, cpn10 is to be administered orally, such as in the form of a tablet or capsule.

In another embodiment, IFN-β and cpn10 are to be administered together by intramuscular or subcutaneous injection.

Cpn10 is preferably provided in purified recombinant synthetic form. Alternatively, cpn10 could be produced by chemical synthesis. However it will be understood by the skilled person that the size of cpn10 may render chemical synthesis a less preferred option.

In this regard, suitable cpn10 nucleotide and amino acid sequences are well known in the art, although for convenience the skilled person is referred to the following mammalian cpn 10 sequences:
(i) human cpn10 (NCBI *Entrez* Accession No. UO7550; Chen *et al.,* 1994, Biochim. Biophys. Acta. **1219**:189-190);
(ii) mouse cpn10 (NCBI *Entrez* Accession No. U09659; Dickson *et al.,* 1994, J. Biol. Chem. **269**:26858-864); and
(iii) rat cpn10 (NCBI *Entrez* Accession No. X71429; Ryan *et al.,* 1994, FEBS Lett. **337**:152-156);

An example of production and purification of recombinant synthetic cpn10 using the pGEX system is provided hereinafter. Another useful approach may be to use eukaryotic expression systems, such as yeast or baculovirus expression systems, for production of recombinant synthetic cpn10. The potential advantages of such systems over bacterial expression is that cpn10 would be produced in a eukaryotic cell and without a modified N-terminus. This may lead to greater specific activity and improved stability. Examples of methods useful for recombinant protein expression can be found in Chapters 5-7 of CURRENT PROTOCOLS IN PROTEIN SCIENCE (Eds. Coligan *et al.;* John Wiley & Sons Inc., 1995-99).

There are currently three sources of IFN-β used in the clinical treatment of MS. These, together with other relevant information are listed in TABLE 1.

Betaseron (or Betaferon), for example, is usually supplied by Schering in dehydrated form together with dextrose and human serum albumin as carrier or diluent. Also included is 0.54% NaCl to act as an aqueous carrier or diluent which rehydrates the dehydrated IFN-β/dextrose/human serum albumin prior to injection.

Suitably, the pharmaceutical composition comprises a pharmaceutically-acceptable carrier or diluent.

By "*pharmaceutically-acceptable carrier or diluent*" is meant a solid or liquid filler, diluent or encapsulating substance which may be safely used in systemic administration. The aforementioned 0.54% NaCl aqueous carrier or diluent which rehydrates dehydrated IFN-β/dextrose/human serum albumin is one example. Other examples of carriers particularly suited to IFN-β are provided in United States Patent Nos. 4,992,271 and 5,643,566.

Depending upon the particular route of administration, a variety of pharmaceutically-acceptable carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline, and pyrogen-free water.

Dosage forms include tablets, dispersions, suspensions, injections, solutions, syrups, troches, capsules, suppositories, aerosols, transdermal patches and the like. These dosage forms may also include controlled release devices or other forms of implants modified to act in this fashion. Controlled release of the therapeutic agent may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

Pharmaceutical compositions of the present invention suitable for administration orally or by injection may be presented as discrete units such as capsules, sachets or tablets, as a powder or granules, or as a solution or a suspension in an aqueous liquid, a nonaqueous liquid, an oil-in-water emulsion or a water-in-oil liquid emulsion. Such compositions may be prepared by any of the methods of pharmacy but all methods include the step of bringing cpn10 and/or IFN-β into association with the carrier. In general, the compositions are prepared by uniformly and intimately admixing the cpn10 and/or IFN-β with liquid carriers or finely divided solid carriers or both, and then, if necessary, shaping the product into the desired presentation.

Also contemplated are carriers which assist pulmonary administration such as via naso-pharyngeal sprays and inhalers as are well known in the art.

It will therefore be apparent that a kit is contemplated for the treatment of MS. In such a case, IFN-β and cpn10 may be provided in dehydrated form together with the carrier or diluent, and both rehydrated prior to injection. Alternatively, the kit may include cpn10 in tablet or capsule form, in which case cpn10 is administered orally while IFN-β is rehydrated in the carrier or diluent for administration by injection.

Also contemplated with regard to said kit are needles, syringes, inhalers, aerosol canisters and the like to assist administration of pharmaceutically-effective amounts of cpn10 and IFN-β.

So that the invention may be understood in more detail, the skilled person is referred to the following non-limiting examples.

### EXAMPLE 1

### General Materials

### 1.1 Animals

Female Lewis rats (JC strain), aged 8-10 weeks, were obtained from the Central Animal Breeding House, The University of Queensland. Female SJL/J mice aged 8 to 12 weeks old were obtained from the Animal Resources Centre, Western Australia. Mature female outbred Quackenbush mice were obtained from Central Animal Breeding House.

All animals were maintained on a continuous supply of mouse/rat pellets and water; in temperature- (22-26°C) and light (12 h light, 12 h dark) controlled rooms. Prior to surgical procedures, rats (average weight 170 g) were anaesthetized with intra-peritoneal anaesthetic mixture (0.2 ml) containing 10 ml ketamine (100 mg/ml), 6.2 ml xylazine (20 mg/ml), 0.8 ml atropine (600 µg/ml) and 10 ml saline (0.9% w/v). All investigations in animals were carried out in accordance with the Australian National Health and Medical Research Committee guidelines with ethical clearance from The University of Queensland Animal Experimentation Ethics Committee.

### 1.2 Recombinant Cpn10 (rcpn10)

Recombinant human cpn10 was prepared using the plasmid pGEX-2T bacterial expression system (Amersham Pharmacia Biotech, Uppsala, Sweden) as described in International Publication No. WO95/15338, which is herein incorporated by reference. Briefly, using a batch technique, the glutathione S-transferase fusion protein was recovered from the cell lysate with glutathione-Sepharose 4B gel (Amersham Pharmacia Biotech), cpn10 cleaved by thrombin [0.05 M Tris-HCL pH 8.0/0.15 M NaCl/2.5 mM CaCl₂ buffer, 1000 units thrombin (Sigma T6884; Sigma-Aldrich, St. Louis, MO, USA); buffer 1] and recovered in the supernatant (sample 1). The gel was then washed with a high salt buffer (0.05 M Tris-HCl pH8.0/2 M NaCl; buffer 2) (sample 2). For inclusion in the lymphocyte proliferation assay (see below), cpn10 was purified on a Resource RPC column (Amersham Pharmacia Biotech); a blank run was used as a control preparation. Protein concentration of the supernatant (cpn10 sample 1) and the high salt wash (cpn10 sample 2) was estimated by the method of Lowry *et al.,* 1951, J. Biol. Chem. **193**:265-75. The purity of the preparations was determined by SDS-PAGE using 15% Tris-Tricine gels (Schagger & von Jagow, 1987, Analytical Biochem., **166**:368-79). The concentration of cpn10 was determined by a double-antibody sandwich ELISA, and bio-activity determined in the rosette inhibition test (Cavanagh & Morton, 1996, Today's Life Sciences, **8**:24-7). The amino acid sequence of cpn10, prepared using the pGEX-2T expression system, is identical to human cpn10 with an additional G-S-M at the N terminus, and the molecule is non-acetylated.

### 1.3. Chemically-synthesized cpn10

Synthetic cpn10 (scpn10) was prepared by stepwise solid phase techniques in an N-terminally acetylated form (Love *et al.,* 1998 In: New Methods for the Study of Biomolecular Complexes, Vol. 510, Series C: Mathematical and Physical Sciences. Ens, Standing & Chernushevich eds. NATO ASI Series, Kluwer Academic Publishers, Netherlands, pp 171-9.

### 1.4 Recombinant IFN-β

Recombinant murine IFN-β (Calbiochem-Novabiochem Corp., La Jolla CA, USA), was supplied in PBS containing 0.1% w/v BSA at 48.4 µg protein (4.5 x 10⁵ units) per mL. Recombinant rat IFN-β (0.1 mg/1 x 10⁵ units; BioSource International, Camarillo, CA USA) was tested in the lymphocyte proliferation assay. Rat IFN-β was dissolved in 1.0 mL distilled water and frozen in aliquots at -70°C, thawed only once. Vehicle alone consisted of PBS with 0.1% w/v BSA (PBS/BSA; Sigma #9418).

### 1.5 Myelin protolipid protein (PLP)

The encephalitogenic PLP peptide, residues 139-151 (HCLGKWLGHPDKF; Greer *et al.,* 1996, J. Immunol. **156**:371-9), was synthesized by step-wise solid phase techniques and purified by reverse-phase HPLC. Purity was determined by electrospray mass spectrometry (≥90% pure).

### EXAMPLE 2

### In vivo experiments with cpn10

### 2.1 Induction of EAE and clinical assessment

Myelin basic protein (MBP) was prepared from guinea pig brains by the method of Deibler *et al*., 1972, Prep. Biochem., **2**:139-165. MBP in 0.9% NaCl w/v (saline) was emulsified in an equal volume of incomplete Freund's adjuvant containing 4 mg/ml *Mycobacterium butyticum.* Under anaesthesia on day 0, rats were inoculated in one hind foot-pad with 0.1 ml emulsion (50 µg MBP/rat). From day 10, rats were weighed daily and clinical signs monitored; the degree of weakness of the tail, hindlimb and forelimb was separately graded on a scale of 0 (no weakness) to 4 (total paralysis) as described by Pender, 1986, J. Neurol. Sci., **75**:317-28). On each day of examination, the total disability in each rat was recorded and results expressed as the mean disability score/group. The rats usually recovered from the disease by day 20, although in some cases a relapse was observed in the following weeks after recovery.

### 2.2 Treatment of EAE with cpn 10

Rats were treated with cpn10 or appropriate control vehicle, starting on the day of inoculation until day 17, in doses ranging from 1 µg to 50 µg, administered every 24 or 48 hrs (see Table 2). Cpn10 sample 1 or buffer 1 were administered to rats without further treatment. Cpn10 sample 2 or buffer 2, following the addition of 1 % normal rat serum, were dialyzed overnight in PBS, before administration. Cpn10 was administered either intraperitoneally (*ip*) or orally with a curved feeding needle (O/F, oral feeding) and weight and clinical score of each rat recorded to day 20. Rats receiving 50 µg/24 hrs were examined up to day 35.

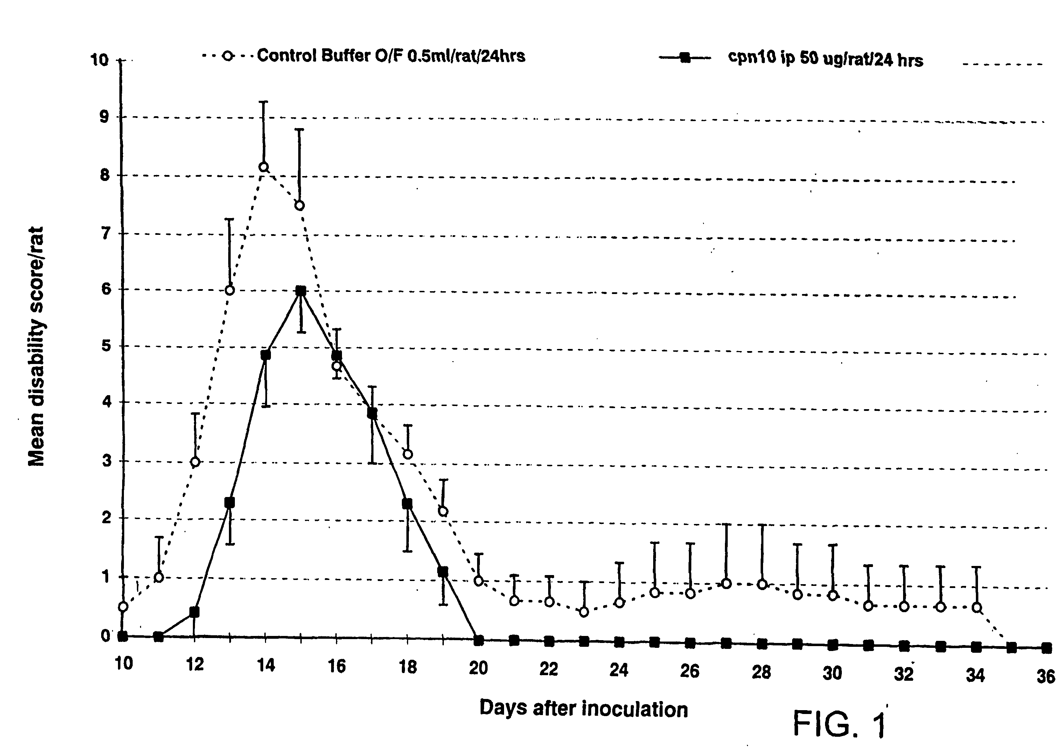

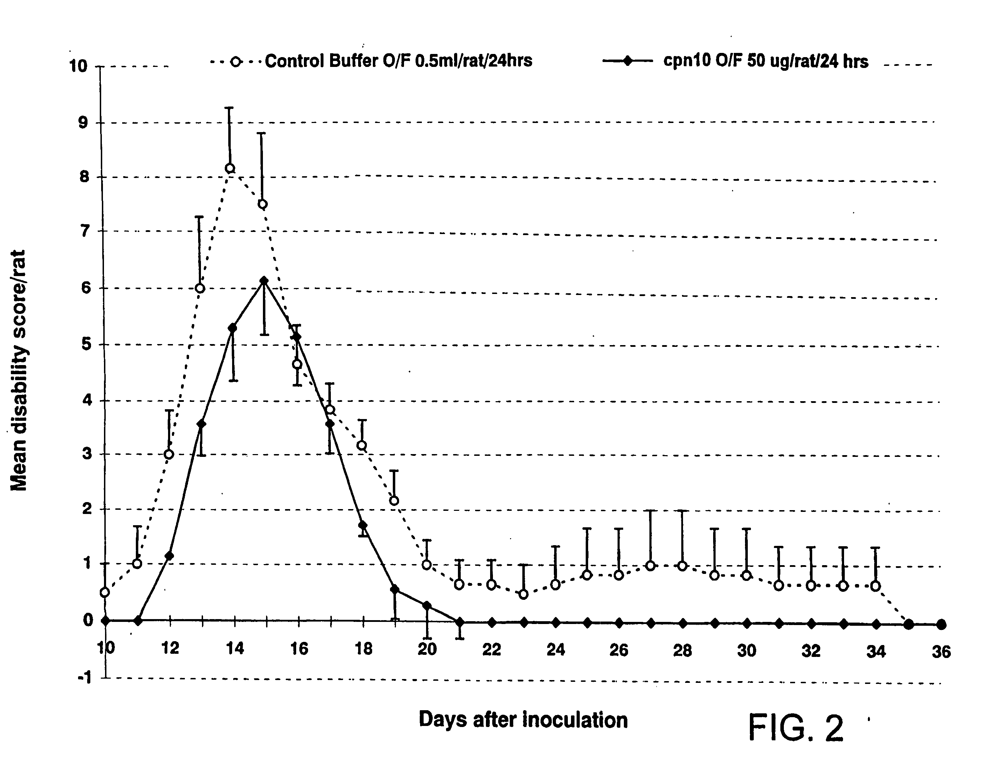

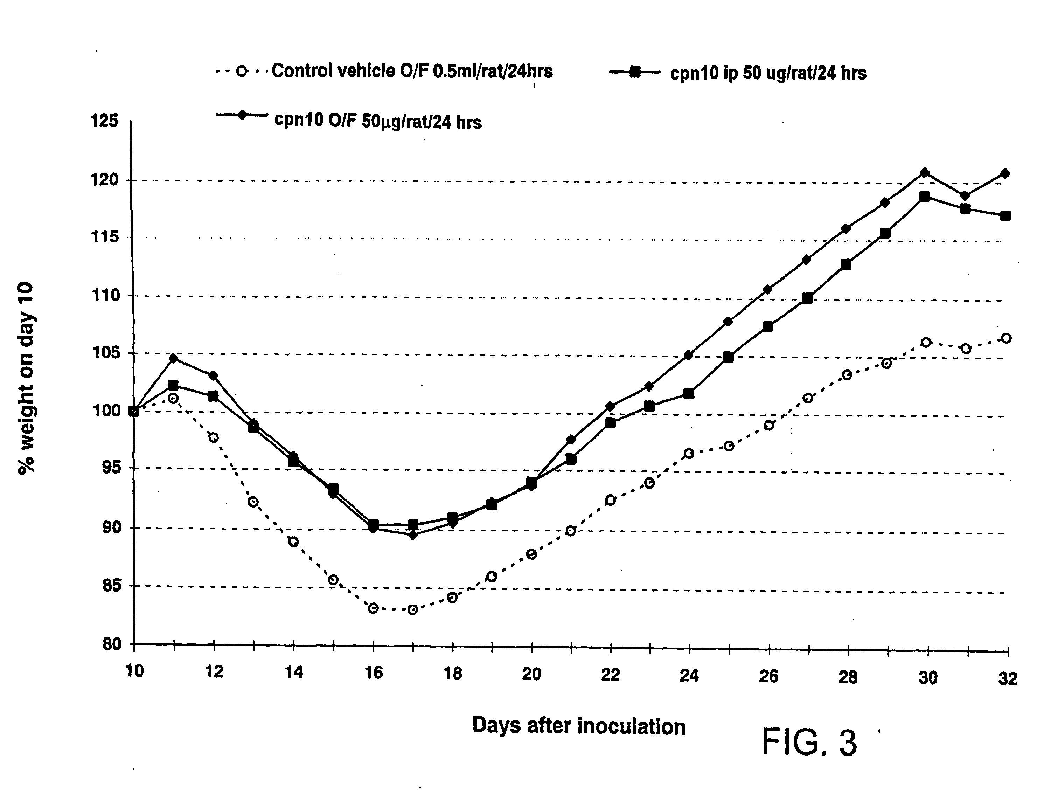

Treatment of EAE rats with cpn10, either intraperitoneally or orally, resulted in a dose-dependent decrease in disability and weight loss between days 10 to 20 (Table 2). In the group of rats receiving 50 µg/day, there was no difference in the total decrease in disability between those rats that received cpn10 i.p. or orally (FIGS. 1 & 2). No incidence of relapse was observed in either of these groups, compared with a low level of relapse in the control group. An expected decrease in weight loss, compared with the control group, was seen (FIG. 3). Once the rats had entered the recovery phase, their gain in weight paralleled that of the control mice (FIG. 3).

At the doses of cpn10 tested, the animals were not completely protected against the clinical signs of EAE but it was not possible to test higher doses due to a limited supply of cpn10. It was also considered that this molecule was not ideal due to the alteration in its N-terminus which interfered with binding to serum carrier proteins. However a significant decrease in clinical score and weight loss was demonstrated with the high dose. No relapses were observed in the cpn10-treated mice on ceasing treatment of day 17 and there was no long-term effect on weight gain. These results are in contrast to those observed following treatment of rats with IFN-β (Ruuls *et al.,* 1996, *supra*).

### 2.3 Cpn10-treated rats showed inhibition of DTH reaction to MBP

Rats were inoculated with MBP, then divided into 4 groups, 4 rats/group. Groups 1 and 4 received vehicle alone, Groups 2 and 3, cpn10 (50 µg/rat/24 hrs) orally and intraperitoneally respectively from day 0 to day 16. On day 15, ear thickness of both ears of rats was measured using an engineer's micrometer (Mitutoyo), then 20 µg MBP in 10 µl saline injected at the base of both ears of Groups 2 to 4. Group 1 rats received saline alone. After 24 hrs, both ears were again measured and ear swelling determined by subtraction.

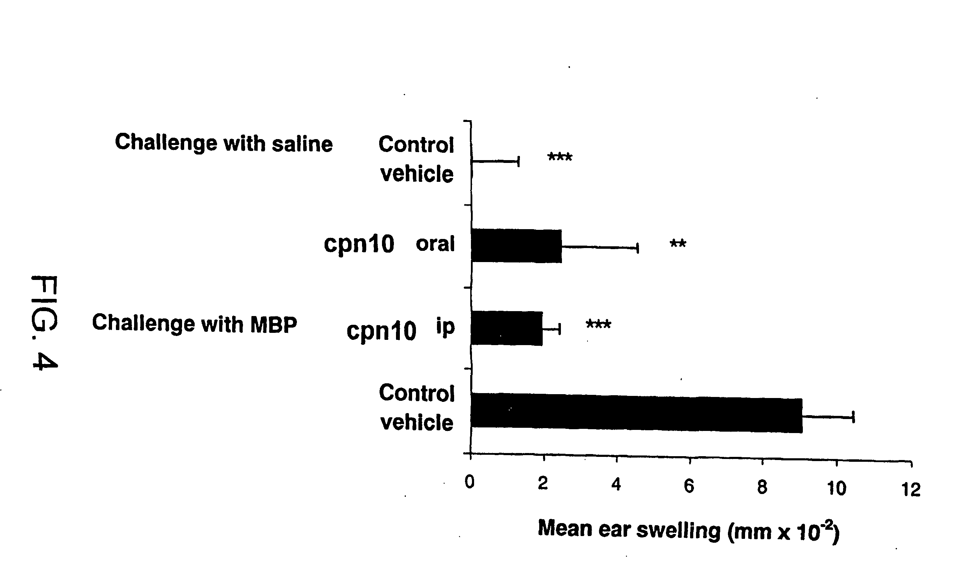

Substantial ear swelling was observed in Group 4 rats receiving control vehicle, challenged with MBP (FIG. 4). Significantly less swelling developed in the rats that received treatment with cpn10 either orally (Group 2) or intraperitoneally (Group 3), prior to MBP challenge. Group 1 rats treated with control vehicle and challenged with saline showed no difference in ear thickness. Visually this difference was very marked as there was a considerable inflamed red area around the base of the ear in Group 4 rats, which was not apparent in Groups 2 and 3. There was no significant difference in the ear swelling between groups 1-3. Similar results were obtained in duplicate experiments.

These results confirm that cpn10, like IFN-β, suppresses the Th1 immune response and show that cpn10, administered orally, is as effective as cpn10 i.p. in this respect.

### 2.4 Total and differential white blood cell (WBC) counts

On day 16 of treatment of EAE rats with vehicle alone, or cpn10 (50 µg/rat/24 hrs), ip or orally (n = 3/group), blood samples were obtained from the tail into EDTA for WBC counts and Wright-Giemsa-stained blood smears prepared for differential counts.

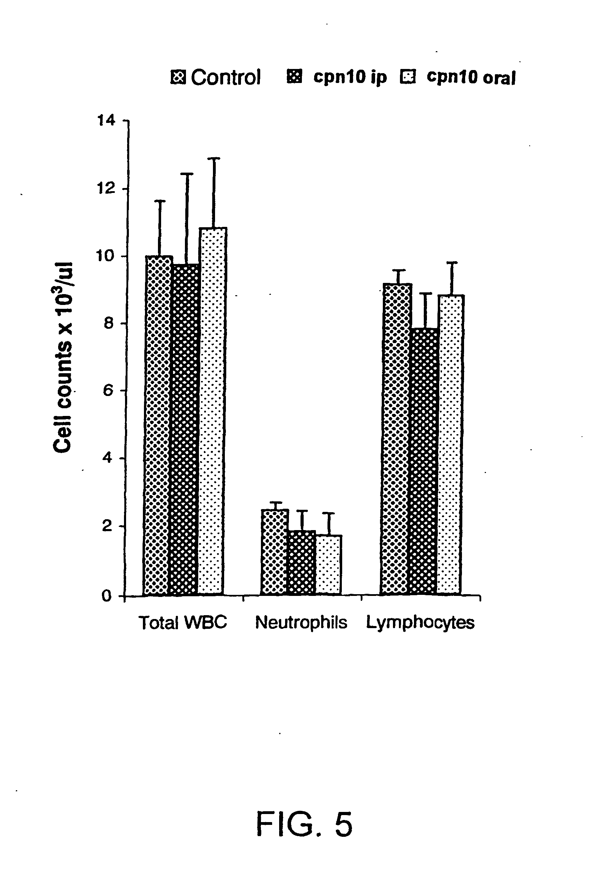

Results are shown in FIG. 5. There was no significant difference in the total WBC, lymphocyte or neutrophil counts between the three groups. Similar results were obtained in duplicate experiments.

These findings, together with those demonstrating that administration of 50 µg cpn10 daily to rats does not effect their weight gain during the recovery period, suggests that cpn10 does not have any very obvious adverse side effects. Once again, this is a characteristic not shared with IFN-β. In humans, IFN-β treatment can cause a marked lymphopoenia and, in mice, Soos *et al.,* 1995, J. Immunol. **155**:2747-53 found a 31.7% depression in lymphocyte counts 12 hrs after injection of IFN-β (10⁵ U/injection).

### EXAMPLE 3

### In vitro experiments with cpn10

### 3.1 Effects of cpn10 on the proliferation of lymph node cells in response to MBP

On day 10 after inoculation, 2 rats were anaesthetized, exsanguinated by cardiac puncture and the popliteal lymph node draining the inoculated limb in each rat removed under sterile conditions. The lymph nodes were chopped finely and suspended in RPMI1640 (ICN, Costa Mesa, CA, USA) containing 2 mM L-glutamine, 25 mM HEPES, 50 µM 2-mercaptoethanol, 1 mM Na-pyruvate, 50 µg/ml gentamycin, 1% heat-inactivated Lewis rat serum (incubation medium). Following removal of debris, the suspended lymphocytes were pooled, washed x 2 and cell concentration adjusted to 4 x 10⁶ cells/ml. Before testing in the proliferation assay, HPLC-purified cpn10 was dialysed for 48 hr (with 1% normal rat serum) against PBS, then transferred into incubation medium on a NAP-5 column (Amersham Pharmacia Biotech) and filter sterilized by passage through a Millex-GV4 0.22 µm Filter Unit (Millipore, Bedford, MA, USA). Control medium (blank run from HPLC) was treated similarly. Concentration of cpn10 in the filtered preparations was confirmed in a double-antibody sandwich ELISA.

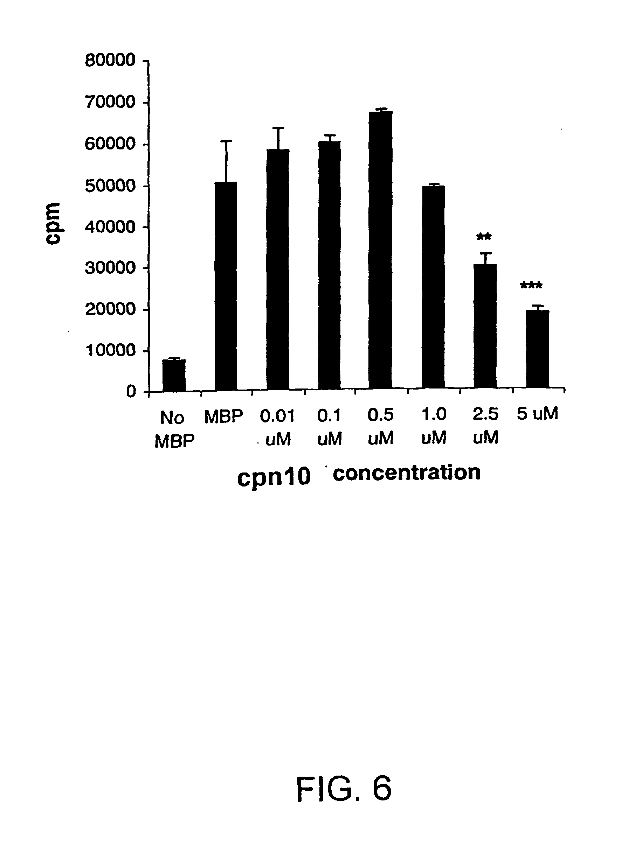

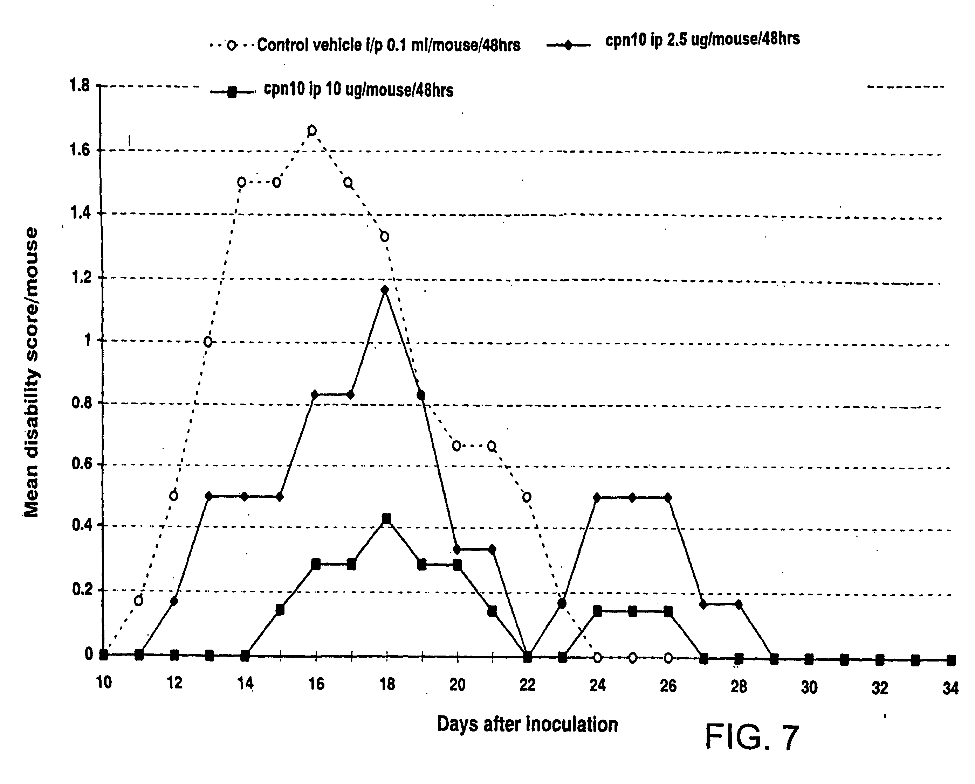

Dilutions of cpn10 were prepared in incubation medium from 100 µg/ml (10.0 µM) to 20 ng/ml (2.0 nM) (see FIG. 6) and 100 µl of each dilution dispensed in triplicate into 96-well flat-bottomed plates (Nunclon ™Δ MicroWell Plates, Nunc, Roskilde, Denmark). MBP (50 µl, 80 µg/ml incubation medium) and prepared lymph node cell suspension (50 µl; 4 x 10⁶ cells/ml) were added to each well. Control wells (6 wells each) contained either (a) cells with MBP but no cpn10 or (b) cells with no MBP nor cpn10. Plates were incubated in a humidified atmosphere at 37°C, 5% CO₂ for 72 hrs. During the last 18 hrs, each well was pulsed with 0.5 µCi [methyl ³H] thymidine (Amersham Pharmacia Biotech) and incorporated radioactivity measured on a scintillation counter (EG&G Wallac, Turku, Finland). Radioactivity incorporated into wells containing cpn10 was compared to that in wells without cpn10. Parallel plates were prepared for assessment of cell viability. After 72 hrs incubation, the supernatant medium was removed, the cells resuspended in 0.1% w/v trypan blue in PBS (20 µl). Cell viability was assessed by trypan blue exclusion.

Cpn10 suppressed the MBP-induced proliferation of lymphocytes in a dose-dependent manner (FIG. 6). Thus, cpn10 can down-regulate MBP-reactive T cells, as does IFN-β (van der Meide *et al.,* 1998, J. Neuroimmunol. **84**:14-23).

### 3.2 Rosette inhibition test

Cpn10 and rat recombinant IFN-β (BioSource International, Camarillo, CA, USA) were tested for activity in the rosette inhibition test using the method previously described (Morton *et al.,* 1976, *supra*; Cavanagh & Morton, 1996, *supra*). Solutions were prepared to contain 1) 1.0 µg cpn10 in 0.5 ml PBS/0.01% BSA w/v (PBS/0.01%BSA) and 2) 1.0 µg rIFN-β in 0.5 ml PBS/0.01%BSA, then transferred into Hank's balanced salt solution/ 0.01% BSA (HBSS/0.01%BSA) on a NAP-5 column (final concentration cpn10 and IFN-β; 1.0 µg/ml). Samples were diluted 10-fold in HBSS/0.01%BSA from 10⁻⁵ to 10⁻¹⁵ and the rosette inhibition titre (RIT) of each dilution determined. The cpn10 titre (log reciprocal sample dilution) was recorded as the highest dilution of a sample to give a positive result in the assay.

The cpn10 sample (1.0 µg/ml) gave a titre of 10⁻¹² in the assay, while the IFN-β sample gave no activity at any concentration.

Cpn10 binds to Th1 cells, releasing genetically restricted suppressor factors, which are active in the rosette inhibition test. These results show that the mechanism whereby IFN-β suppresses the Th1 immune response differs from that of cpn10.

In the rat model, the results have shown that treatment with cpn10:-
(i) decreases the disability and weight loss of animals with EAE;
(ii) suppresses the DTH reaction *in vivo*; and
(iii) down-regulates the activity of encephalitogenic (MBP-reactive) cells *in vitro.*

Thus, like IFN-β, cpn10 will suppress the Th1 response. However the means by which this response is down-regulated appears to differ.

Cpn10 is active in the rosette inhibition test, a measure of its ability to release specific genetically-restricted suppressor factors from CD4+ T cells. IFN-β has no activity in this assay.

Administration of cpn10 to rats does not affect the total lymphocyte count, whereas Soos *et al*., 1995, *supra* have shown that IFN-β treated mice develop a marked lymphopoenia. This has also been observed in patients receiving IFN-β treatment. Unlike treatment with IFN-β, treatment with cpn10 does not have any long term effect on the well-being of the rats as assessed by weight gain subsequent to treatment.

These results have shown that the mode of action of IFN-β and cpn10 differ but both can suppress the Th1 immune response and, in doing so, modify the symptoms of EAE. A mouse model of EAE was developed to determine if combined complementary therapy between IFN-β and cpn10 in the treatment of EAE could be achieved.

### EXAMPLE 4

### EAE induction in mice

EAE was induced in SJUJ mice by sc injection in the flank with 100 µg PLP p139-151 and 400 µg *Mycobacterium tuberculosis* H37Ra (Difco Laboratories, Detroit, MI, USA) in an emulsion of water and complete Freund's adjuvant (Sigma-Aldrich, St. Louis, MO, USA; Greer *et al.,* 1996, *supra*). Each mouse was also injected iv on d 0 and d 3 with 0.3 µg Pertussis toxin (*Bordetella pertussis,* List Biological Laboratories, Inc, CA, USA) in 0.3 ml 0.9% w/v NaCl (saline). Daily from d 8, mice were assessed clinically as follows: 0 = no disease; 1 = decreased tail tone and slightly clumsy gait; 2 = tail atony and/or moderately clumsy gait and/or poor righting ability; 3 = limb weakness; 4 = limb paralysis, 5 = moribund state. Mice were examined until d 42 or d 60 (see Table 3). On each day of examination, the mean disability score/mouse in each treatment group was determined.

Over the examination period, the total mean disability score/mouse/group was calculated. This value was also expressed as total mean disability score in the primary attack (d 8 to d 21) and during the period of relapse (d 22 to d 42 or d 60; see Table 4) that occurred within the examination period. In two groups of mice (Experiments #4 and #5, see Table 3), the period of examination was extended from d 60 to d 68 and total mean disability score from d 61 to d 68 recorded.

### EXAMPLE 5

### Effect of cpn10 therapy upon EAE in mice

Groups of mice (n = 10/group) were treated with recombinant cpn10 (rcpn10) in doses of 2.5 µg, 5 µg, 10 µg or 20 µg/mouse, administered *ip* every other day. Treatment commenced on the day of inoculation (day 0) or at the time of onset of disease (day 8) and continued to day 18 or 20 (see Table 3). Period of examination is shown in Table 3. With the different dose regimens, the total mean disability score in the group receiving rcpn10 was compared with that in the group receiving vehicle alone, tested concurrently (Mann-Whitney Rank Sum test, Sigma Stat 2.0, Jandel Scientific).

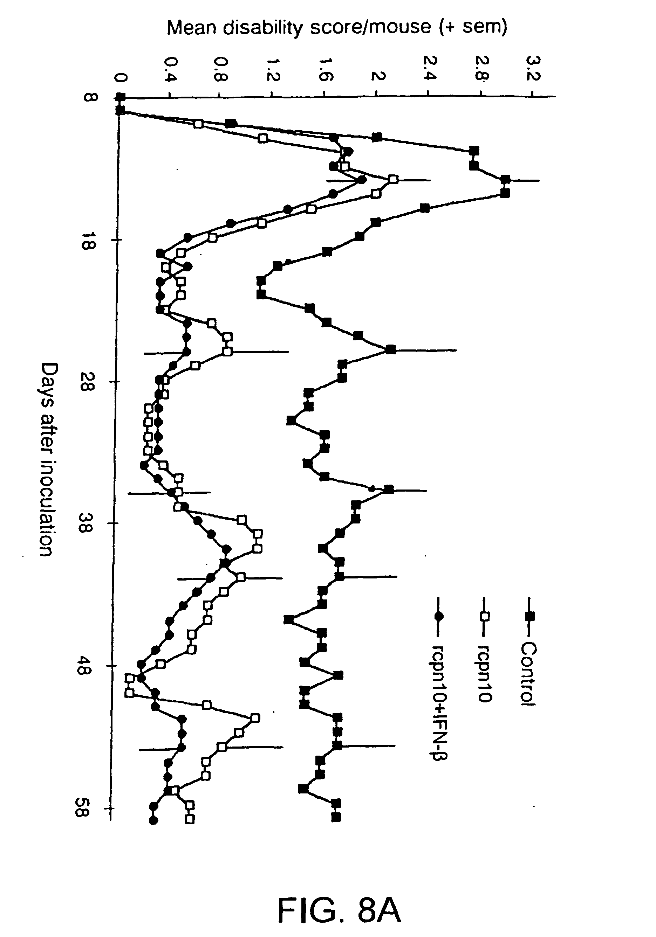

Following inoculation with PLP peptide 139-151, mice (10/group) developed a chronic relapsing form of EAE. Onset of clinical signs appeared between day 8 and day 10, peaked at d 14 to 16 and decreased by day 21 (Figure 8A). Mice underwent an incomplete recovery and subsequently had further episodes of clinical disease. Treatment with 2.5 µg, 5 µg or 10 µg rcpn10 every other day from d 0 to day 18 or day 20 significantly suppressed total disability over the examination period, compared with the group receiving vehicle alone (Table 3). While 20 µg rcpn10/dose gave suppression of disease, the results were not significantly different from those in the group receiving vehicle (Table 3). No difference in response in the treatment groups was observed if treatment was initiated at the time of onset of disease (day 8), rather than at the time of inoculation (day 0; Table 3). After day 60, the mice in the rcpn10 treated groups developed clinical signs of EAE with total mean disability score from day 61 to day 68 not significantly different from that in the groups of mice receiving vehicle alone (data not shown).

### EXAMPLE 6

### Combined INF-β and cpn10 therapy

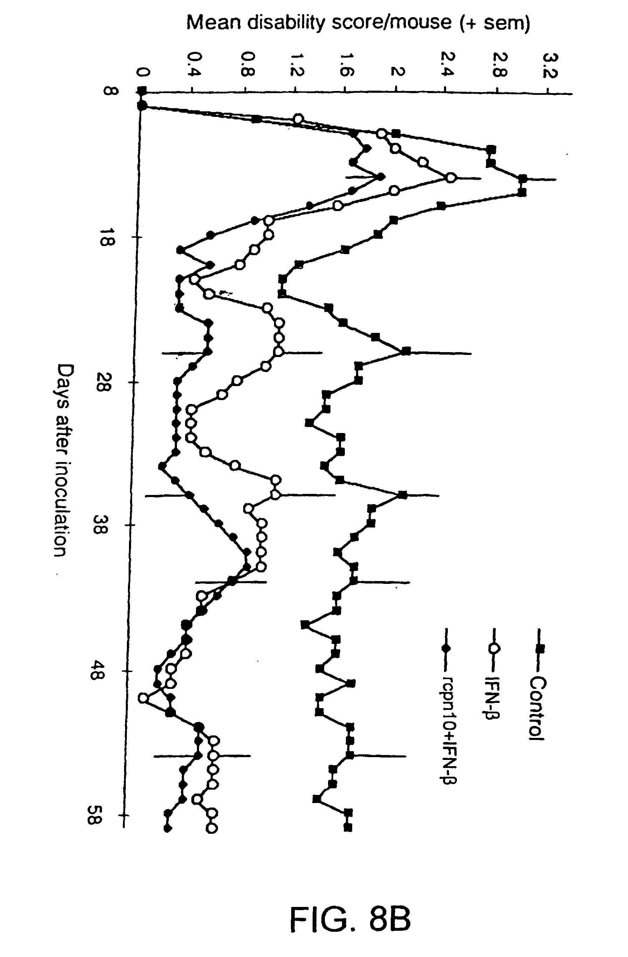

To determine if rcpn10 and IFN-β act co-operatively in suppression of clinical signs of EAE, groups of mice were treated with rcpn10 and IFN-β, separately and together (Figures 8A and 8B). Sub-optimal doses of rcpn10 and IFN-β were selected so that a co-operative effect could readily be observed, if present. Four groups of mice (n = 10/group) were tested. On the day of inoculation, Groups 2 and 4 received 2.5 µg rcpn10 *ip* every other day from day 0 to day 20 and Groups 1 and 3 received vehicle. IFN-β was diluted to 10⁵ units/ml in saline and mice in Groups 3 and 4 received 5,000 units (0.5 µg; Yu *et al.,* 1996, *supra*) sc in 50 µl saline every other day from day 10 to day 20. Mice in Groups 1 and 2 received 50 µl 0.1 % BSA in PBS, diluted ¼ in saline. On day 18 and day 20, all mice received treatment with antihistamine (Pyrilamine, Sigma-Aldrich), 10 µg/g body weight *ip* (Jose *et al.,* 1994, J. Exp. Med. **179**:881-887), to prevent anaphylaxis from administration of BSA carrier present in the IFN-β and control vehicle preparations. Mice were examined daily as described above until day 60 and results in the groups receiving cpn10, IFN-β, cpn10+IFN-β were compared with the group receiving vehicle alone (Mann-Whitney Rank Sum test). This experiment was performed in duplicate.

Treatment of EAE mice with 2.5 µg rcpn10 every other day from day 0 to day 20 suppressed the total mean disability score during the examination period of day 8 to day 60 when compared with mice receiving vehicle alone (Figure 8A, Table 4). In light of these results, the present inventors compared the efficacy of rcpn10 and IFN-β, given that IFN-β is a well-known treatment of MS and also displays activity against EAE (Yu *et al*., 1996, *supra*). Administration of 5,000 units IFN-β every other day from day 10 to day 20 did not give a significant suppression during the same examination period (Figure 8B, Table 4). However, if administered together, the combination rcpn10 and IFN-β gave greater suppression, when compared to the control group, than either of the reagents given alone (Figures 8A, 8B, Table 4). The suppressive effect on disability was more apparent during the period of relapse (day 22 to day 60) than in the primary attack (day 8 to day 21). While neither cpn10 nor IFN-β treatment alone gave significant suppression during the primary attack, the combination treatment was effective (Figure 9, Table 4). During the period of relapse, suppression of the total mean disability score, compared with the control group, was enhanced by use of combination therapy (Table 4). Similar results were obtained in the duplicate experiment (data not shown).

During preliminary experiments (data not shown), approximately 50% mice died 10 to 12 days (*ie* day 20 - day 22), after delivery of BSA sc either in the IFN-β preparation or in the control vehicle. This was considered to be due to anaphylactic shock from sensitisation to BSA. In the experiments reported above, mice were treated with antihistamine on day 18 and day 20 (Jose *et al*., 1994, supra) and no further deaths occurred.

### EXAMPLE 7

### Histology of mice treated with recombinant cpn10 or vehicle

On day 14 after inoculation, two mice from the group receiving 2.5 µg recombinant cpn10/mouse *ip* every other day and two from the group receiving vehicle alone were anaesthetised and perfused with Karnovsky's fixative (McCombe *et al*., 1992, *supra*). The mice selected for perfusion from each group had a disability score representative of mean of the group on that day. Spinal cords were removed and embedded in Epox 8112 and semi-thin sections from the cervical (C5), thoracic (T6), lumbar (L4) and sacral (S2) spinal cord and the cauda equina stained with toluidine blue. Sections were examined for evidence of inflammation and demyelination.

Sections from multiple levels of the spinal cord from a control and a rcpn10-treated mice are shown in Figure 10. In the control mouse, inflammation in the sub-pial regions and in the perivascular regions within the parenchyma can be seen. There was evidence of demyelination, more severe in the sacral and lumbar spinal cord than in the cervical cord. In the rcpn10 treated mouse, less inflammation, particularly in the parenchyma, and less demyelination can be demonstrated.

### EXAMPLE 8

### Proliferation of lymph node cells in vitro in response to MBP

Activity of IFN-β, recombinant cpn10 and chemically-synthesized cpn10 (scpn10) in an *in vitro* lymphocyte proliferation assay was determined. Prior to use, rcpn10 (200 µg/ml) and scpn10 (100 µg/ml) with 1% autologous rat serum were dialysed for 3 days against phosphate buffered saline (PBS; 0.05 M sodium phosphate buffer pH 7.4/0.15 M sodium chloride), transferred into incubation medium (RPMI1640 containing 2 mM L-glutamine, 25 mM HEPES, 50 µM 2-mercaptoethanol, 1 mM Na-pyruvate, 50 µg/ml gentamycin, 1% heat-inactivated autologous rat serum) on a NAP-5 column (Amersham Pharmacia Biotech) and filter sterilized by passage through a Millex-GV4 0.22 µm Filter Unit (Millipore, Bedford, MA, USA). Control vehicle was treated similarly. MBP-EAE was induced in Lewis rats by inoculation with MBP. On day 10, popliteal lymph nodes draining the inoculated limb in 2 rats were removed and a cell suspension (4 x 10⁶/ml) prepared in incubation medium. Lymph node cells (2 x 10⁵/well) were tested in triplicate in 96-well flat-bottomed plates in the presence of MBP (20 µg/ml final concentration) and IFN-β or cpn10 at final concentrations ranging from 0.1 - 50 µg/ml as shown in Figure 11. Control wells (6 wells each) contained (a) cells with MBP but no cpn10 and (b) cells with no MBP and no cpn10. Plates were incubated at 37°C, 5% CO₂ for 72 h. During the last 18 h, each well was pulsed with 0.5 µCi [*methy*/⁻³H]thymidine (Amersham Pharmacia Biotech) and incorporated radioactivity measured. Results were expressed as stimulation index, *ie*, mean of wells with MBP/mean of wells without MBP. Stimulation index with various dilutions of cpn10 was compared with the stimulation index without cpn10 (Student's *t* test).

Following 72 hr in culture, rcpn10, scpn10 and IFN-β all suppressed MBP-induced proliferation of cells from lymph nodes of MBP-EAE rats (Figure 11). IFN-β was the most potent, with 50% inhibition of cell proliferation at a concentration of 1.9 µg/ml (96 nM), compared with 11 µg/ml sCpn10 (1.1 µM) and 28 µg/ml rcpn10 (2.8 µM) to achieve this level of inhibition.

### EXAMPLE 9

### Rosette inhibition test, the bioassay for cpn10

Scpn10, rcpn10 and IFN-β were tested for activity in the rosette inhibition test, the bioassay for cpn10 (Cavanagh & Morton, 1996, *supra*). rcpn10 and scpn10 (50 µg) were diluted 10-fold in Hank's balanced salt solution/0.01% BSA (HBSS/BSA) from 10⁻⁵ to 10⁻¹⁵. IFN-β (0.5 µg/ml) was diluted 10-fold in HBSS/BSA to 10⁻¹³. The rosette inhibition titre (RIT) of each dilution was determined using spleen cells from Quackenbush mice. Limiting dose (log reciprocal sample dilution) was recorded as the highest dilution of a sample to give a positive result in the assay.

In these experiments, rcpn10 and scpn10 behaved identically in the rosette inhibition test (Figure 12). In contrast, IFN-β had no activity in this assay at any concentration.

### EXAMPLE 10

### Cpn10 prevents antigen-induced anaphylactic death in mice

In light of the experiments discussed in Example 6, mouse deaths resulting from what appeared to be anaphylactic shock were further investigated. Four groups of mice were inoculated with PLP p139-151. At day 0 two groups received cpn10 and two groups control vehicle. At day 10 one of each cpn10 group received IFN-β or PBS/BSA, while one of each control vehicle group received IFN-β or PBS/BSA.

As shown in Table 5, between day 8 and day 12 after commencement of IFN-β in PBS/BSA or PBS/BSA injections, 4/9 and 6/10 mice died in the groups receiving BSA but no cpn10, while 3/10 and 0/9 mice died in the groups receiving BSA and cpn10. It is believed that these mice died of anaphylaxis from administration of BSA carrier protein. It was concluded that the mice receiving cpn10 were protected against anaphylactic shock cased by immunization with BSA, whereas IFN-β did not afford this protection. There was also reduced inflammation at the site of administration when cpn10 was administered.

### EXAMPLE 11

### General Discussion

The results presented here demonstrated that cpn10 suppressed clinical signs of EAE induced with myelin proteolipid protein in SJL/J mice. This model is a chronic relapsing form of EAE that mimics the clinical course of MS in humans. Patients with MS have enhanced T cell responses to PLP peptides (Greer *et al.,* 1997, Brain **120** 1447-60), further suggesting that EAE induced with PLP is a good model of MS. The most effective doses to suppress disability during the examination period were 5 µg and 10 µg, delivered every other day. Higher doses were less effective. Similar reduction in efficacy at higher doses has been observed previously in a study demonstrating that cpn10 delivered locally at the site of the graft could prolong the survival time of allogeneic skin graft in rats. The suppressive effect of cpn10 in the EAE mice was more apparent during the period of relapse than during the primary attack. The reason for greater suppression of disability during the period of relapse than in the primary attack may be that cpn10 augments natural suppressor mechanisms, active during this period, or that relapses have a different pathophysiology from the first episode. During the later episodes of chronic EAE, there may be spread of the immune response to other antigens and enhanced antibody responses, so that the pathogenesis of later episodes differ from that of the primary attack. This study also showed that cpn10 and IFN-β co-operated in their ability to suppress EAE. Administration of cpn10 and IFN-β together give a greater suppression of disability than either substance administered alone. While this was particularly evident in the period of relapse of the disease, at all times cpn10 and IFN-β co-operated in their ability to suppress disease and an antogonistic effect was not observed.

Cpn10 and IFN-β reacted differently in the rosette inhibition test and the lymphocyte proliferation assay suggesting that they differ in their mechanism of action. The rosette inhibition test is the assay by which EPF was first discovered (Morton *et al.,* 1976, *supra*) and has remained the bioassay for EPF and cpn10. The action of EPF in the rosette inhibition test is mediated by lymphokines, EPF-S₁ and EPF-S₂, sequentially induced following binding of EPF to CD4+ cells in mice and humans (Rolfe *et al.,* 1988, *supra;* Rolfe *et al.,* 1989, *supra)*. These lymphokines were designated suppressor factors due to their ability to suppress the delayed-type hypersensitivity reaction (Rolfe *et al.*, 1988, *supra*). They are genetically restricted in their activity. The restriction of EPF-S₁ (Mr 14 - 18,000) has been mapped to the I region of the murine H-2 and to HLA-DR in humans, while that of the EPF-S₂ (Mr ~55 000) is localised to the Igh region (Rolfe *et al.,* 1988, *supra;* Rolfe *et al.,* 1989, *supra*; Rolfe *et al.,* 1995, *supra*). Suppression of clinical signs following treatment with rcpn10 was maintained until day 60 despite treatment ceasing after the primary attack. This suggests the induction of long-term suppressive activity. IFN-β is not active in the rosette inhibition test, showing that it does not use the same suppressor-inducer pathway in downregulating lymphocyte activity.

IFN-β is considerably more active than cpn10 in its ability to suppress the proliferation of MBP-activated lymphocytes *in vitro* in response to MBP. Both IFN-β (Arnason, 1993, Neurology **43**:641-643) and cpn10 suppress IFN-γ production by activated T-cells. IFN-β inhibits IFN-γ secretion, augments defective suppressor activity in MS patients and inhibits class II major histocompatibility complex (MHC) antigen expression induced by IFN-γ on the surface of antigen-presenting cells (The IFNB Multiple Sclerosis Study Group, 1993, Neurology **43**: 655-661). Cpn10 binds to sub-populations of CD4+ cells, CD8+ cells and monocytes. Binding of cpn10 to a sub-population of activated CD4+ T cells results in suppression of IFN-γ production. The means by which cpn10 induces immunomodulatory effects and how this related to its ability to suppress disability in EAE has yet to be determined.

These studies have shown that cpn10 can suppress chronic EAE and can augment the ability of IFN-β to suppress the clinical signs of EAE in mice. Crucially, the differing mechanisms of action of cpn10 and IFN-β elucidated by the present inventors have opened a new outlook on these agents as co-operative treatments of EAE and MS. If cpn10 and IFN-β acted via similar immunosuppressive mechanisms, as indeed was suggested by the literature, then there could be no co-operative or synergistic effect observable in the EAE model. In contrast, their differing mechanisms of action and the EAE data presented herein, have provided a rationale for the proposition that cpn10 and IFN-β will be a useful combined therapy of MS in humans.

IFN-β is used extensively as a disease-modifying treatment for multiple sclerosis (MS) in humans. It reduces the clinical relapse rate, delays time of onset of sustained progress of disability and reduces the number of new MRI lesions in patients with relapsing-remitting multiple sclerosis (European Study Group on Interteronβ-1b, 1998, Lancet **352**:1491-1497). However some patients do experience sustained progression of disability within 2 years of commencement of treatment suggesting a need for further study to investigate the efficacy of IFN-β with that of IFN-β plus other promising therapies.

In summary, the animal model results presented herein suggest that cpn10 is a potential candidate for use in combination with IFN-β in the treatment of MS in humans. Firstly, cpn10 appears to synergize with IFN-β in reducing EAE/MS symptoms. Secondly, a pronounced reduction in EAE relapse following cessation of cpn10 and IFN-β combined therapy suggests that this may be a useful treatment should IFN-β administration be temporarily ceased due to side-effects. Thirdly, cpn10 seems to reduce the likelihood of anaphylactic reactions to carrier protein antigens (such as BSA) typically present in pharmaceutical compositions which include IFN-β.

It will be appreciated that the invention described in detail herein is susceptible to modification and variation by persons skilled in the art, such modifications and variations nevertheless falling within the scope of the invention set forth herein.

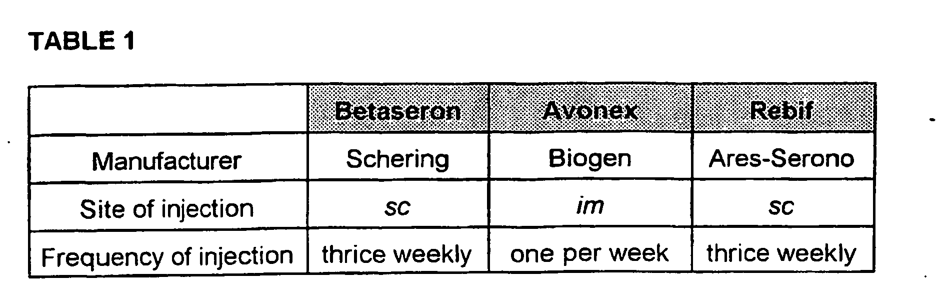

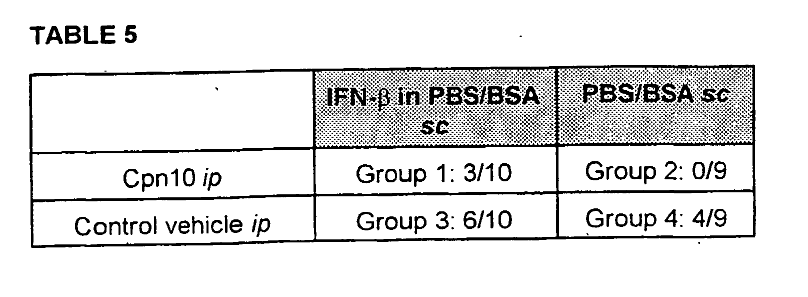

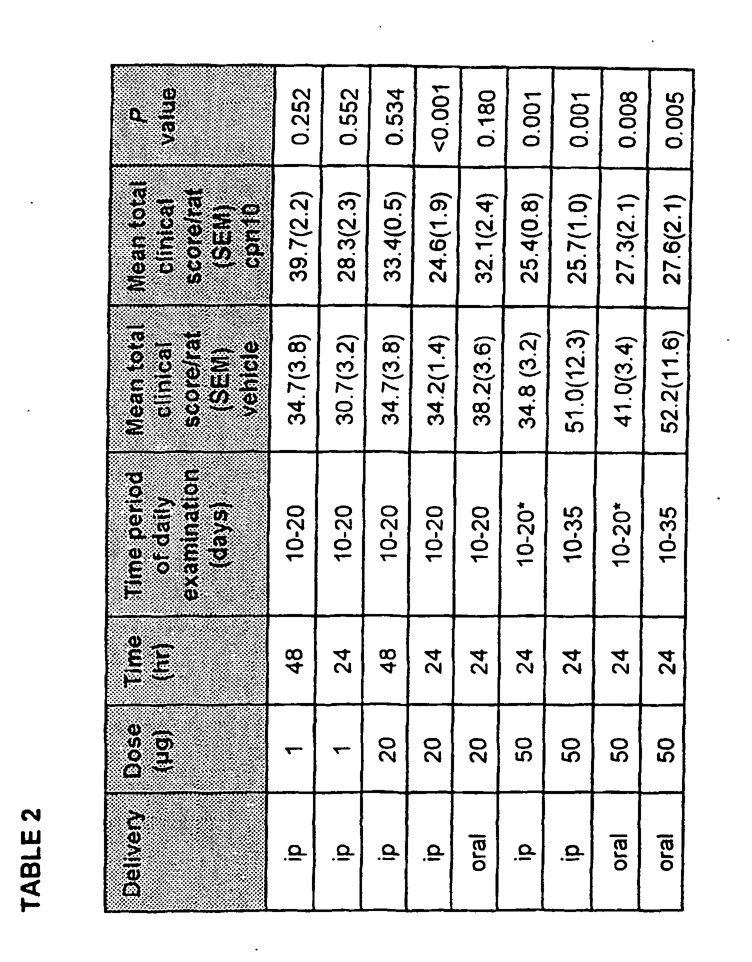

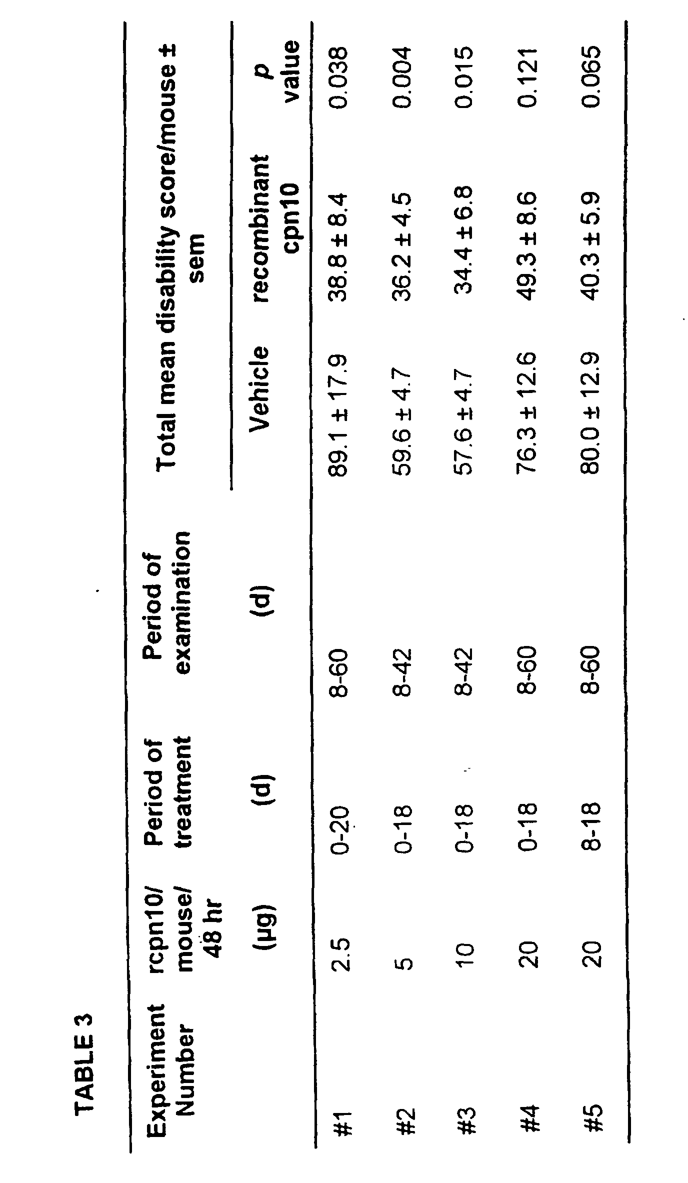

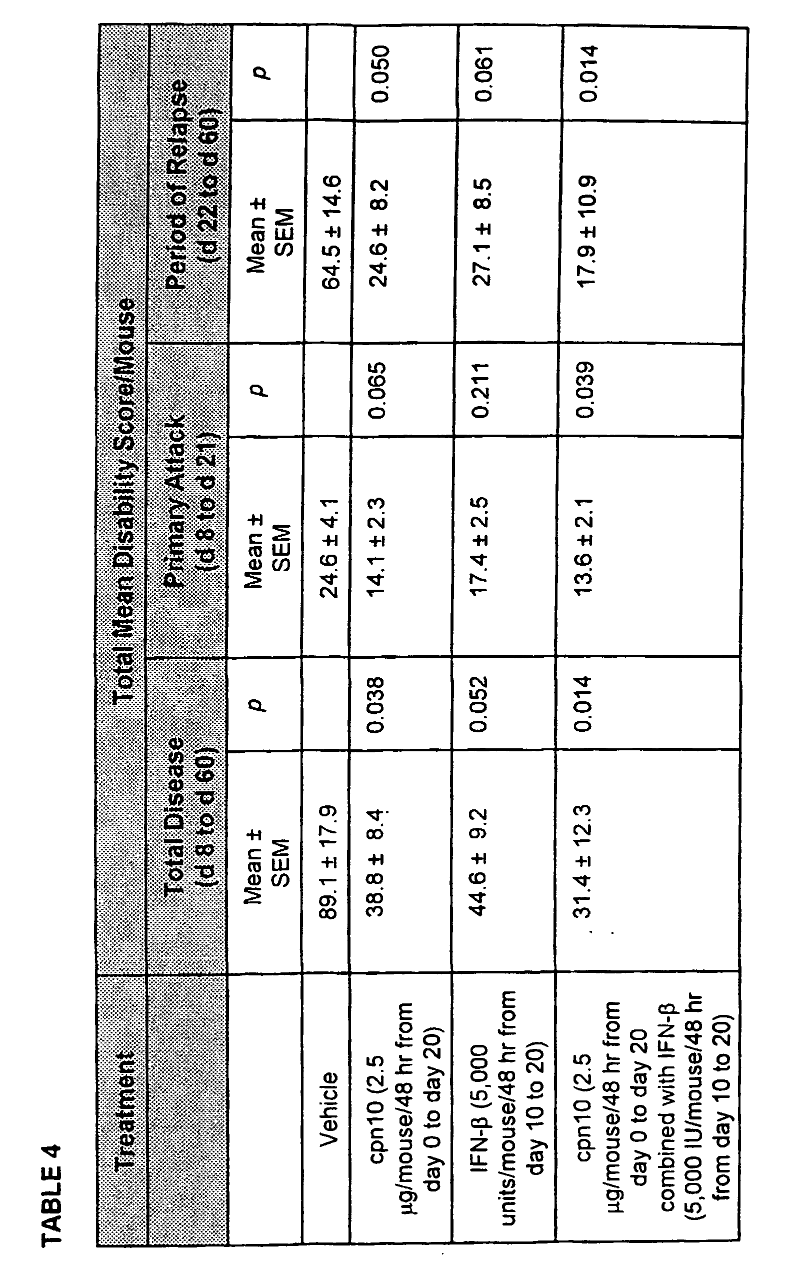

## Claims

1. Use of Chaperonin 10 (cpn10) and β-interferon (IFN-β) in the manufacture of a medicament for treating multiple sclerosis (MS).

2. Use according to claim 1 wherein the medicament is for treatment to prevent relapse of MS.

3. Use according to Claim 1 or Claim 2, wherein IFN-β and cpn10 are for administration together.

4. Use according to Claim 1 or Claim 2, wherein IFN-β and cpn10 are for administration separately.

5. Use according to Claim 3, wherein IFN-β and cpn10 are for administration by injection.

6. Use according to Claim 4, wherein the cpn10 is for administration orally.

7. Use according to Claim 4 or Claim 6, wherein the IFN-β is for administration by injection.

8. Use according to Claim 1 or Claim 2, wherein the medicament comprises 5-60 mg of cpn10.

9. Use according to Claim 8, wherein the medicament comprises 10-30 mg of cpn10.

10. Use according to Claim 1 or Claim 2, wherein the medicament comprises 1-10 Million International Units (MIU) of IFN-β.

11. Use according to Claim 10, wherein the medicament comprises 4-6 MIU of IFN-β.

12. A pharmaceutical composition for treating MS, said composition comprising a pharmaceutically-effective amount of Chaperonin 10 (cpn10) and β-interferon (IFN-β), and a pharmaceutically-acceptable carrier or diluent.

13. The composition of Claim 12, wherein the pharmaceutically-effective amount of cpn10 and IFN-β comprises 5-60 mg of cpn10.

14. The composition of Claim 13, wherein the pharmaceutically-effective amount of cpn10 and IFN-β comprises 10-30 mg of cpn10.

15. The composition of Claim 12, wherein the pharmaceutically-effective amount of cpn10 and IFN-β comprises 1-10 MIU of IFN-β.

16. The composition of Claim 15, wherein the pharmaceutically-effective amount of cpn10 and IFN-β comprises 4-6 MIU of IFN-β.

17. A kit comprising a pharmaceutically-effective amount of Chaperonin 10 (cpn10) and β-interferon (IFN-β) and a pharmaceutically-acceptable carrier or diluent.

18. The kit of Claim 17, wherein said IFN-β is in dehydrated form, which in use, is rehydrated by said pharmaceutically-acceptable carrier or diluent.

19. The kit of Claim 18, wherein said cpn10 is in dehydrated form and in use, is rehydrated by said pharmaceutically-acceptable carrier or diluent.

20. The kit of Claim 17 or Claim 18, wherein said cpn10 is in tablet or capsule form.

21. The kit of Claim 17, wherein the pharmaceutically-effective amount of cpn10 and IFN-β comprises 5-60 mg of cpn10.

22. The kit of Claim 21, wherein the pharmaceutically-effective amount of cpn10 and IFN-β comprises 10-30 mg of cpn10.

23. The kit of Claim 17, wherein the pharmaceutically-effective amount of cpn10 and IFN-β comprises 1-10 MIU of IFN-β.

24. The kit of Claim 23, wherein the pharmaceutically-effective amount of cpn10 and IFN-β comprises 4-6 MIU of IFN-β.

25. A product containing Chaperonin 10 (cpn10) and β-interferon (IFN-β) as a combined preparation for simultaneous, separate or sequential use in treatment of multiple sclerosis (MS).

## Patentansprüche

1. Verwendung von Chaperonin 10 (cpn10) und β-Interferon (IFN-β) bei der Herstellung eines Medikaments zur Behandlung von multipler Sklerose (MS).

2. Verwendung nach Anspruch 1, worin das Medikament zur Behandlung zur Unterbindung eines Rückfalls von MS verwendet wird.

3. Verwendung nach Anspruch 1 oder Anspruch 2, worin IFN-β und cpn10 gemeinsam zu verabreichen sind.

4. Verwendung nach Anspruch 1 oder Anspruch 2, worin IFN-β und cpn10 getrennt voneinander zu verabreichen sind.

5. Verwendung nach Anspruch 3, worin IFN-β und cpn10 mittels Injektion zu verabreichen sind.

6. Verwendung nach Anspruch 4, worin das cpn10 oral zu verabreichen ist.

7. Verwendung nach Anspruch 4 oder Anspruch 6, worin das IFN-β mittels Injektion zu verabreichen ist.

8. Verwendung nach Anspruch 1 oder Anspruch 2, worin das Medikament 5-60 mg cpn10 umfasst.

9. Verwendung nach Anspruch 8, worin das Medikament 10-30 mg cpn10 umfasst.

10. Verwendung nach Anspruch 1 oder Anspruch 2, worin das Medikament 1-10 MIU (Million Internationale Einheiten) IFN-β umfasst.

11. Verwendung nach Anspruch 10, worin das Medikament 4-6 MIU IFN-β umfasst.

12. Pharmazeutische Zusammensetzung zur Behandlung von MS, worin die Zusammensetzung eine pharmazeutisch wirksame Menge Chaperonin 10 (cpn10) und β-Interferon (IFN-β) und ein(en) pharmazeutisch annehmbaren/s Träger oder Verdünnungsmittel umfasst.

13. Zusammensetzung nach Anspruch 12, worin die pharmazeutisch wirksame Menge von cpn10 und IFN-β 5-60 mg cpn10 umfasst.

14. Zusammensetzung nach Anspruch 13, worin die pharmazeutisch wirksame Menge von cpn10 und IFN-β 10-30 mg cpn10 umfasst.

15. Zusammensetzung nach Anspruch 12, worin die pharmazeutisch wirksame Menge von cpn10 und IFN-β 1-10 MIU IFN-β umfasst.

16. Zusammensetzung nach Anspruch 15, worin die pharmazeutisch wirksame Menge an cpn10 und IFN-β 4-6 MIU INF-β umfasst.

17. Set, umfassend eine pharmazeutisch wirksame Menge von Chaperonin 10 (cpn10) und β-Interferon (IFN-β) und ein(en) pharmazeutisch annehmbaren/s Träger oder Verdünnungsmittel.

18. Set nach Anspruch 17, worin das IFN-β in dehydratisierter Form vorliegt und bei Verwendung durch den/das pharmazeutisch annehmbare(n) Träger oder Verdünnungsmittel rehydratisiert wird.

19. Set nach Anspruch 18, worin das cpn10 in dehydratisierter Form vorliegt und bei Verwendung durch den/das pharmazeutisch annehmbare(n) Träger oder Verdünnungsmittel rehydratisiert wird.

20. Set nach Anspruch 17 oder Anspruch 18, worin das cpn10 in Form von Tabletten oder Kapseln vorliegt.

21. Set nach Anspruch 17, worin die pharmazeutisch wirksame Menge von cpn10 und IFN-β 5-60 mg cpn10 umfasst.

22. Set nach Anspruch 21, worin die pharmazeutisch wirksame Menge von cpn10 und IFN-β 10-30 mg cpn10 umfasst.

23. Set nach Anspruch 17, worin die pharmazeutisch wirksame Menge von cpn10 und IFN-β 1-10 MIU IFN-β umfasst.

24. Set nach Anspruch 23, worin die pharmazeutisch wirksame Menge von cpn10 und IFN-β 4-6 MIU IFN-β umfasst.

25. Produkt, das Chaperonin 10 (cpn10) und β-Interferon (IFN-β) in Form eines Kombinationspräparats zur gleichzeitigen, separaten oder sequenziellen Verwendung bei der Behandlung von multipler Sklerose (MS) enthält.

## Revendications

1. Utilisation de Chap éronine 10 (cpn10) et de β-interféron (IFN-β) dans la fabrication d'un médicament pour le traitement de la sclérose en plaques (MS).

2. Utilisation selon la revendication 1 où le médicament est pour un traitement pour prévenir la rechute de MS.

3. Utilisation selon la revendication 1 ou la revendication 2, où IFN-β et cpn10 sont pour une administration ensemble.

4. Utilisation selon la revendication 1 ou la revendication 2, où IFN-β et cpn10 sont pour une administration séparément.

5. Utilisation selon la revendication 3, où IFN-β et cpn10 sont pour une administration par injection.

6. Utilisation selon la revendication 4, où cpn10 est pour une administration orale.

7. Utilisation selon la revendication 4 ou la revendication 6, où IFN-β est pour une administration par injection.

8. Utilisation selon la revendication 1 ou la revendication 2, où le médicament comprend 5-60mg de cpn10.

9. Utilisation selon la revendication 8, où le médicament comprend 10-30mg de cpn10.

10. Utilisation selon la revendication 1 ou la revendication 2, où le médicament comprend 1-10 millions d'unités internationales (MUI) de IFN-β.

11. Utilisation selon la revendication 10, où le médicament comprend 4-6 MUI de IFN-β.

12. Composition pharmaceutique pour le traitement de SP, ladite composition comprenant une quantité pharmaceutiquement efficace de Chap2 ronine 10 (cpn10) et β-interféron (IFN-β) et un support ou diluant pharmaceutiquement acceptable.

13. Composition de la revendication 12, où la quantité pharmaceutiquement efficace de cpn10 et IFN-β comprend 5-60mg de cpn10.

14. Composition de la revendication 13, où la quantité pharmaceutiquement efficace de cpn10 et IFN-β comprend 10-30mg de cpn10.

15. Composition de la revendicat ion 12, où la quantité pharmaceutiquement efficace de cpn10 et IFN-β comprend 1-10 MUI de IFN-β.

16. Composition de la revendication 15, où la quantité pharmaceutiquement efficace de cpn10 et IFN-β comprend 4-6 MUI de IFN-β.

17. Kit comprenant une qu antité pharmaceutiquement efficace de Chapéronine 10 (cpn10) et β-interféron (IFN-β) et un support ou un diluant pharmaceutiquement acceptable.

18. Kit de la revendication 17, où ledit IFN-β est sous forme déshydratée qui, en utilisation, est réhydraté par ledit support ou diluant pharmaceutiquement acceptable.

19. Kit de la revendication 18, où cpn10 est sous forme déshydratée et, en utilisation, est réhydraté par ledit support ou diluant pharmaceutiquement acceptable.

20. Kit de la revendication 17 ou de la revendication 18, où cpn10 est sous forme de comprimés ou de capsules.

21. Kit de la revendication 17, où la quantité pharmaceutiquement efficace de cpn10 et IFN-β comprend 5-60mg de cpn10.

22. Kit de la revendication 21, où la quantité pharmaceutiquement efficace de cpn 10 et IFN-β comprend 10-30mg de cpn10.

23. Kit de la revendication 17, où la quantité pharmaceutiquement efficace de cpn10 et IFN-β comprend 1-10 MUI de IFN-β.

24. Kit de la revendication 23, où la quantité pharmaceutiquement efficace de cpn 10 et IFN-β comprend 4-6 MUI de IFN-β.

25. Produit comprenant de la Ch apéronine 10 (cpn10) et du β-interféron (IFN-β) sous la forme d'une préparation combinée pour usage simultané séparé ou séquentiel dans le traitement de la sclérose en plaques (SP).
